# EUROPEAN PATENT SPECIFICATION

(11) **EP 1 121 129 B1**
(45) Date of publication and mention of the grant of the patent: **03.09.2008**
(21) Application number: 99951438.3
(22) Date of filing: 13.09.1999
(51) Int. Cl.: A61K 31/50, A61K 31/505, A61K 31/42, A61K 31/00, A61K 45/06

(54) **METHOD FOR TREATING DIABETES EMPLOYING AN aP2 INHIBITOR AND ASSOCIATED COMBINATIONS**
METHODE ZUR BEHANDLUNG VON DIABETES DIE EINEN AP2-INHIBITOR UND DAZUGEHÖRIGE KOMBINATIONEN VERWENDET
PROCEDE DE TRAITEMENT DE DIABETES A L'AIDE DE L'INHIBITEUR AP2 ET COMBINAISON ASSOCIEE

(30) Priority: 17.09.1998 US 100677 P
(43) Date of publication of application: 08.08.2001
(73) Proprietor: Bristol-Myers Squibb Company, Princeton, NJ 08543-4000 (US)
(72) Inventor: ROBL, Jeffrey, A., Newtown, PA 18940 (US); PARKER, Rex, A., Titusville, NJ 08560 (US); BILLER, Scott, A., Hopewell, NJ 08525 (US); JAMIL, Haris, Newton, PA 18940 (US); JACOBSON, Bruce, L., Mercerville, NJ 08619 (US); KODUKULA, Krishna, Princeton, NJ 08540 (US)
(74) Representative: Vossius & Partner
(86) International application number: PCT/US1999/020946
(87) International publication number: WO 2000/015229

(56) References cited:
- WO-A-91/06538
- US-A- 5 218 124
- KLETZIEN ET AL.: "Induction of aP2 by an Insulin Sensitizing Agent" J.CEL.BIOCHEM.SUPPL., vol. 15, no. B, 1991, page 70 XP002209109
- MELKI ET AL.: "Expression of the Adipocyte Fatty Acid- Binding Protein in Streptozotin- Diabetes: Effects of Insulin Deficiency and Supplementation" J.LIPID RES., vol. 34, no. 9, 1993, pages 1527-34, XP001094445
- CHEMICAL ABSTRACTS, vol. 126, 1996, Columbus, Ohio, US; abstract no. 17417, HOTMISLIGIL G.S. ET AL.: 'Uncoupling of Obesity from Insulin Resistance Through a Targeted Mutation in aP2, the Adipocyte Fatty Acid Binding Protein' XP002953082 & SCIENCE vol. 274, no. 5291, 1996, WASHINGTON, D.C., pages 1377 - 1379
- LALONDE J.M. ET AL: 'X-ray Crystallographic Structures of Adipocyte Lipid-Binding Protein Complexed with Palmitate and Hexadecanesulfonic Acid. Properties of Cavity Binding Sites' BIOCHEMISTRY vol. 33, 1994, pages 4885 - 4895
- SULSKY R. ET AL BIOORGANIC & MEDICINAL CHEMISTRY LETTERS 2006, DOI:10.1016/J.BMCL.2006.12.044,

## Description

### Field of the Invention

The present invention relates to the use of an aP2 inhibitor alone or in combination with another type antidiabetic agent for the manufacture of a medicament for treating diabetes, especially Type II diabetes, as well as hyperglycemia, hyperinsulinemia, obesity, hypertriglyceridemia and related diseases.

### Background of the Invention

Fatty acid binding proteins (FABPs) are small cytoplasmic proteins which bind to fatty acids such as oleic acids which are important metabolic fuels and cellular regulators. Dysregulation of fatty acid metabolism in adipose tissue is a prominent feature of insulin resistance and the transition from obesity to non-insulin dependent diabetes mellitus (NIDDM or Type II diabetes).

aP2, an abundant 14.6 KDa cytosolic protein in adipocytes, and one of a family of homologous intracellular fatty acid binding proteins (FABPs), is involved in the regulation of fatty acid trafficking in adipocytes and mediates fatty acid fluxes in adipose tissue. G.S. Hotamisligil et al, "Uncoupling of Obesity from Insulin Resistance Through a Targeted Mutation in aP2, the Adipocyte Fatty Acid Binding Protein", Science, Vol. 274, Nov. 22, 1996, pp. 1377-1379, report that aP2-deficient mice placed on a high fat diet for several weeks developed dietary obesity, but, unlike control-mice on a similar diet, did not develop insulin resistance or diabetes. Hotamisligil et al conclude that "aP2 is central to the pathway that links obesity to insulin resistance" (Abstract, page 1377).

DIALOG ALERT DBDR928 dates January 2, 1997, Pharmaprojects No. 5149 (Knight-Ridder Information) discloses that a major drug company "is using virtual screening techniques to identify potential new antidiabetic compounds." It is reported that "the company is screening using aP2, a protein related to adipocyte fatty acid binding protein."

### Description of the Invention

In accordance with the present invention, the use of a therapeutically effective amount of a drug which inhibits aP2 (aP2 inhibitor) for the manufacture of a medicament is provided for treating diabetes, especially Type II diabetes, and related diseases such as insulin resistance, hyperglycemia, hyperinsulinemia, elevated blood levels of fatty acids or glycerol, obesity and hypertriglyceridemia

In addition, in accordance with the present invention, a use is provided for treating diabetes and related diseases as defined above and hereinafter, wherein a therapeutically effective amount of a combination of an aP2 inhibitor and another type antidiabetic agent is administered to a human patient in need of treatment.

Furthermore, in accordance with the present invention, a novel antidiabetic combination is provided which is formed of a drug which inhibits aP2 and another type antidiabetic agent which functions by a mechanism other than by inhibiting aP2. The aP2 inhibitor will be employed in a weight ratio to the antidiabetic agent (depending upon its mode of operation) within the range from about 0.01:1 to about 100:1, preferably from about 0.5:1 to about 10:1.

The aP2 inhibitors suitable for use in the method of the invention are compounds which bind to the aP2 protein and inhibits its function and/or its ability to bind free fatty acids. The compounds will preferably contain less than 60 carbon atoms, more preferably less than 45 carbon atoms, and will contain less than 20 heteroatoms, more preferably less than 12 heteroatoms. They contain a hydrogen bond donator or acceptor group, preferably acidic in nature, which includes, but is not limited to, CO₂H, tetrazole, SO₃H, PO₃H, P(R)(O)OH (where R is lower alkyl or lower alkoxy), OH, NHSO₂R' or CONHSO₂R' (where R' is lower alkyl), and thiazolidindione, and interacts (directly or through an intervening water molecule), either by ionic or hydrogen bonding interactions, with one, two, or three of the three amino acid residues, designated as Arg 106, Arg 126 and Tyr 128 in human aP2, within the aP2 protein.

The compounds suitable for use herein preferably contain an additional substituent, preferably hydrophobic in nature, which include the following groups: alkyl, cycloalkyl, aryl, heteroaryl, cycloheteroalkyl, benzo-fused aryl and heteroaryl, and their substituted counterparts. Especially preferred are aryl and substituted aryl groups. More especially preferred is phenyl and halo or methyl substituted phenyl.

The hydrophobic substituent binds to (in) and/or interacts with a discrete pocket within the aP2 protein defined roughly by the amino acid residues Phe 16, Tyr 19, Met 20, Val 23, Val 25, Ala 33, Phe 57, Thr 74, Ala 75, Asp 76, Arg 78 in human aP2. The through space distance from the hydrogen bond donor/acceptor group and the additional substituent group is within the distance of about 7 to about 15 Angstroms.

The above compounds may be employed in the form of pharmaceutically acceptable salts thereof and prodrug esters thereof.

### Brief Description of Figure

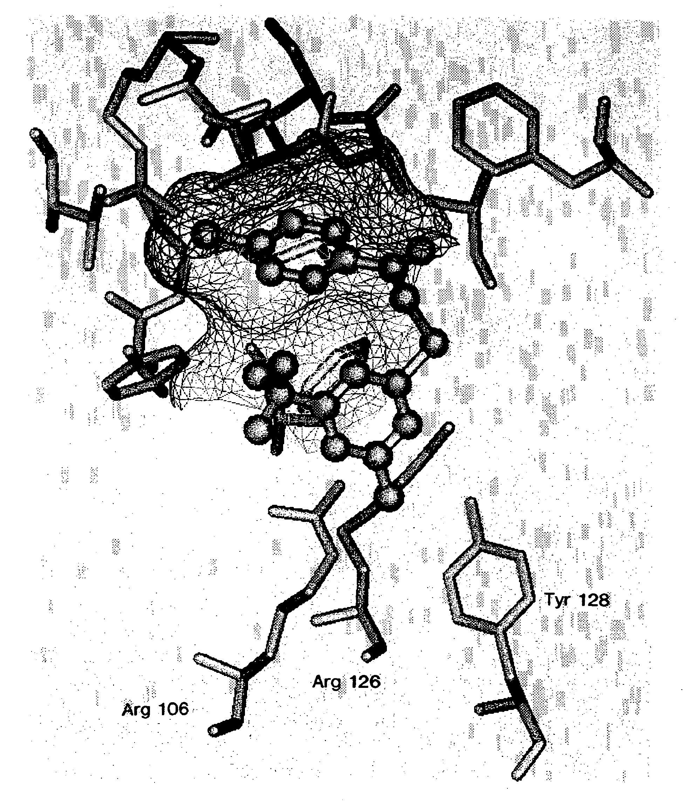

The accompanying Figure is a computer generated image of a partial X-ray structure of compound XVIA (described hereinafter) bound to human aP2.

### Detailed Description of the Invention

Examples of aP2 inhibitors suitable for use herein include compounds which include an oxazole or analogous ring. Thus, U.S. Patent No. 5,218,124 to Failli et al discloses compounds, which have activity as aP2 inhibitors and thus suitable for use herein, which include substituted benzoylbenzene, biphenyl- and 2-oxazole-alkanoic acid derivatives having the following structure:

I A (CH₂)ₙO-B

wherein
A is a group having the formula 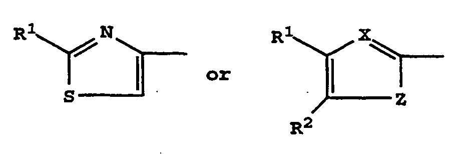 wherein
X is -N- or 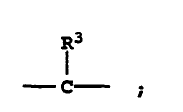 Z is 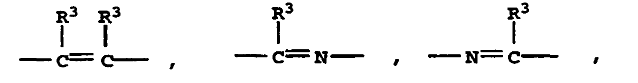 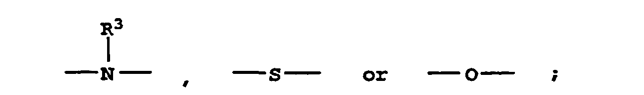 R¹ is hydrogen, lower alkyl or phenyl;
R² is hydrogen or lower alkyl; or
R¹ and R² taken together form a benzene ring, with the proviso that when X is -N-, Z is other than 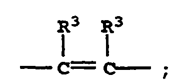 R³ is hydrogen or lower alkyl;
n is 1-2;
B is 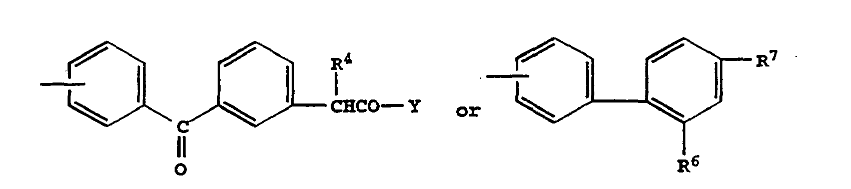 wherein
Y is OR⁵ or N(OH)R⁸;
R⁴ and R⁵ are each, independently, hydrogen or lower alkyl;
R⁶ is hydrogen, halo or nitro;
R⁷ is 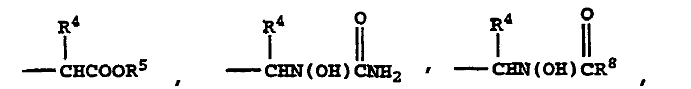 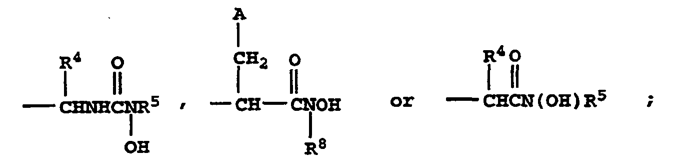 R⁸ is lower alkyl;
m is 0-3;
and the pharmacologically acceptable salts thereof.

The grouping A embraces, inter alia, 5- or 6-membered unsaturated nitrogen, sulfur or oxygen containing mono- or benzofused-heterocycles, optionally substituted with lower alkyl or phenyl. The foregoing definition embraces the following heterocyclic moieties; furyl, pyrrolyl, thienyl, oxazolyl, thiazolyl, imidazolyl, pyridyl, pyrazinyl, pyrimidinyl, benzofuranyl, benzothienyl, benzothiazolyl, indolyl, benzoxazolyl, quinazolinyl, benzimidazolyl, quinoxalinyl, quinazolinyl and the like.

Preferred are the examples where A is defined as above and B is
and R⁷ is

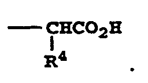

In another embodiment of the present invention, compounds which have activity as aP2 inhibitors suitable for use herein are disclosed in U.S. Patent No. 5,403,852 to Barreau et al (which is incorporated herein by reference) which are oxazole derivatives and have the structure 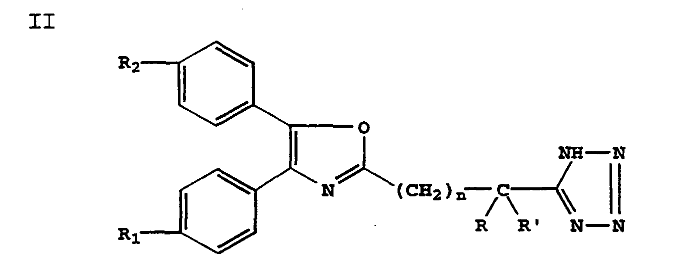 in which;
R and R' are identical or different and represent a hydrogen atom or an alkyl radical containing 1 or 2 carbon atoms,
R₁ and R₂ are identical or different and represent hydrogen or halogen atoms or alkyloxy radicals in which the alkyl portion contains 1 to 4 carbon atoms in a straight or branched chain, and
n equals 3 to 6,
as well to their salts , to their isomers where they exist and to pharmaceutical compositions containing them.

In addition, other compounds which have activity as aP2 inhibitors suitable for use in the method of the invention are compounds disclosed in U.S. Patent No. 4,001,228 to Mattalia (which is incorporated herein by reference) which are 2-thiol-4,5-diphenyloxazole S-derivatives which have the structure 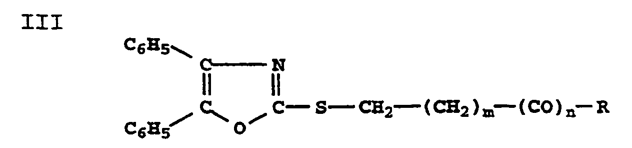 wherein m is 0, 1 or 2, n is 1 and R represents hydroxy, alkoxy or amino. Also included within the scope of this invention are salts of the compounds of formula III above, particularly pharmaceutically acceptable addition salts thereof.

Preferred are S-(4,5-diphenyloxazol-2-yl)-mercaptocarboxylic acids of the formula: 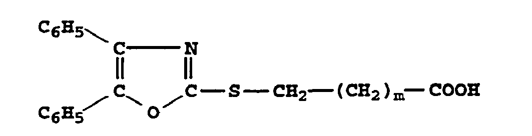 wherein m is 0, 1 or 2, and pharmaceutically acceptable lower alkyl esters and salts thereof.

In another embodiment of the present invention, compounds which have activity as aP2 inhibitors suitable for use herein are disclosed in U.S. Patent No. 4,051,250 to Dahm et al (the disclosure of which is incorporated herein by reference) which discloses azole derivatives of the structure 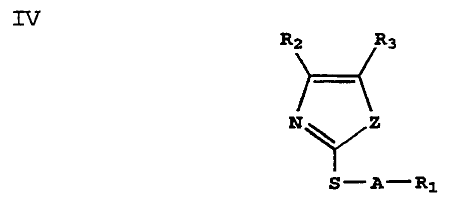 wherein R₁ is carboxyl, esterified carboxyl or other functionally modified carboxyl group; R₂ and R₃ each are aryl of up to 10 carbon atoms; A is CₙH₂ₙ in which n is an integer from 1 to 10, inclusive; and Z is O or S, and the physiologically acceptable salts thereof.

Preferred are preferred compounds as disclosed in the Dahm et al patent.

In still another embodiment of the invention, compounds which have activity as aP2 inhibitors suitable for ue herein are disclosed in U.S. Patent No. 5,380,854 to Romine et al and are phenyl-heterocyclic oxazole derivatives which have the structure 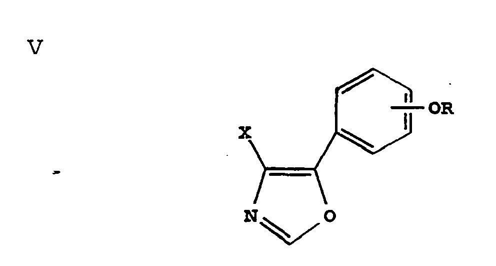 X is 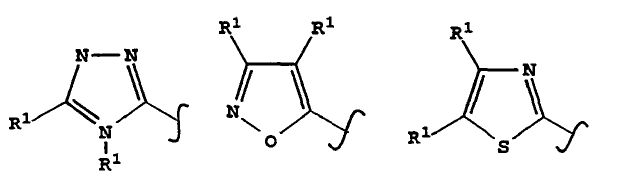 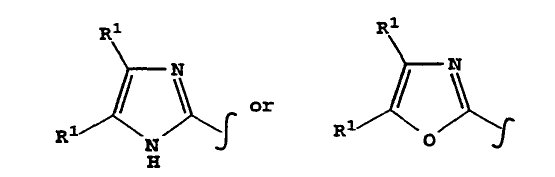 R is CH₂R²;
R¹ is Ph or Th;
R² is 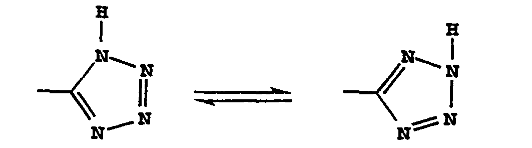 CO₂R³, and
R³ is H, or C₁-C₄ lower alkyl;
or pharmaceutically acceptable salt thereof.

Preferred are the compounds where R is CH₂CO₂H and 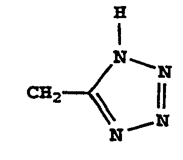 or its tautomer and R¹ is Ph.

In yet another embodiment of the method of the invention, compounds which have activity as aP2 inhibitors suitable for use herein are disclosed in PCT application WO 95/17393 which are diaryloxazole derivatives having the structure 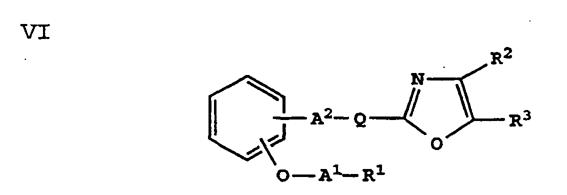 wherein R¹ is carboxy or protected carboxy,
R² is aryl which may have suitable substituent(s),
R³ is aryl which may have suitable substituent(s),
A¹ is lower alkylene,
A² is bond or lower alkylene and
-Q- is 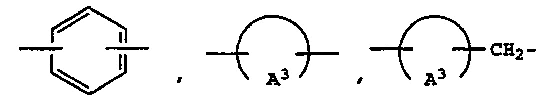 or 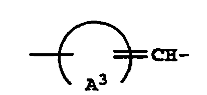 (in which 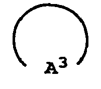 is cyclo (lower)alkane or
cycle(lower)alkene, each of which may have suitable substituent(s)).

Preferred are the preferred compounds of WO 95/17393 as illustrated by the working Examples thereof.

Another embodiment of compounds which have activity as aP2 inhibitors suitable for use herein are disclosed in U.S. Patent No. 5,362,879 to Meanwell (the disclosure of which is incorporated herein by reference) which are 4,5-diphenyloxazole derivatives having the structures 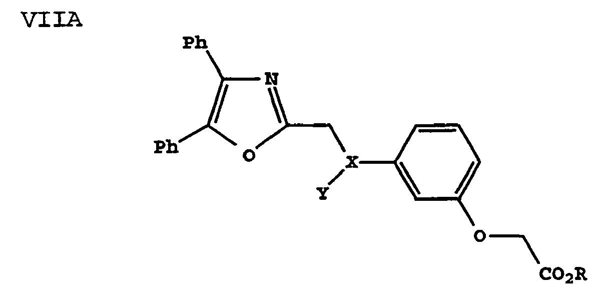 wherein
R' is H or C₁-C₅ lower alkyl,
X is N or CH,
Y is H or CO₂R¹, or COR²,
R¹ is C₁-C₅ lower alkyl, or phenylmethyl, and
R² is C₁-C₅ alkyl; 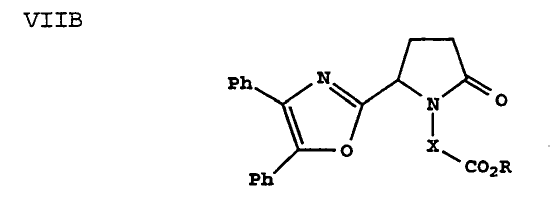 wherein
R is H or C₁-C₅ lower alkyl,
X is (CH₂)ₙ or para or meta substituted phenyl
wherein the substituent is OR²,
R² is C₁-C₅ alkyl, and
n is an integer of 4 to 8,
and pharmaceutically acceptable salts thereof.

Preferred are the preferred compounds of the Meanwell patent as illustrated by the working Examples thereof.

In still another embodiment of the present invention, compounds which have activity as aP2 inhibitors suitable for use herein are disclosed in U.S. Patent No. 5,187,188 to Meanwell (the disclosure of which is incorporated herein by reference) which are oxazole carboxylic acid derivatives having the structure 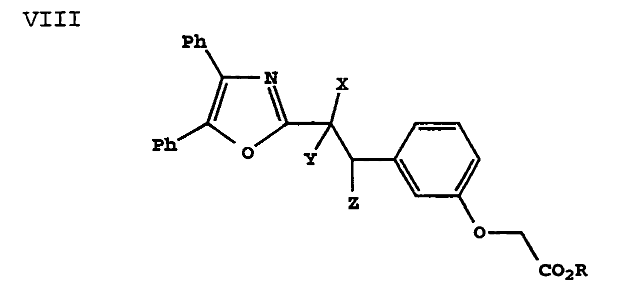 wherein
Y and Z are independently hydrogen or together form a bond;
X is CN, CO₂R¹ or CONR²R³;
R and R¹ are independently or together H, Na, or C₁-C₅ lower alkyl;
R² and R³ are independently or together H, or C₁-C₅ lower alkyl;
or alkali metal salt thereof.

Preferred are the preferred compounds of the above Meanwell patent as illustrated by the working Examples thereof.

In another embodiment of the invention, compounds which have activity as aP2 inhibitors suitable for use herein are disclosed in U.S. Patent No. 5,348,969 to Romine et al which are phenyloxazolyloxazole derivatives having the structure 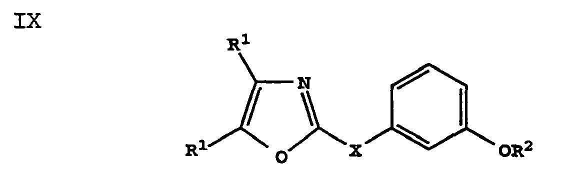 wherein
X is 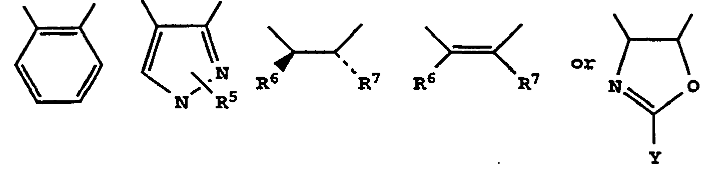 Y is CH₃, Ph, or OH, provided that when Y is OH, the compound exists in the keto-enol tautaumerism form 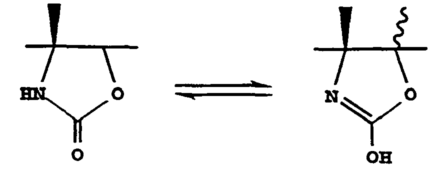 R¹ is Ph or Th;
R² is CH₂R³;
R³ is CO₂R⁴;
R⁴ is H or C₁-C₅ lower alkyl;
R⁵ is H or CH₃; R⁶ is OHCHN or H₂N; and
R⁷ is H or OH;
or pharmaceutically acceptable salt thereof.

Preferred are the preferred compounds as delineated in the Romine et al patent and in the working Examples thereof, especially where X is 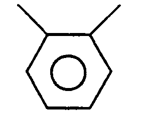 and R² is CH₂CO₂H.

In addition, compounds which have activity as aP2 inhibitors which may be employed herein include those disclosed in U.S. Patent No. 5,262,540 to Meanwell and are 2-(4,5-diaryl)-2-oxazolyl substituted phenoxyalkanoic acids and esters having the strucutre 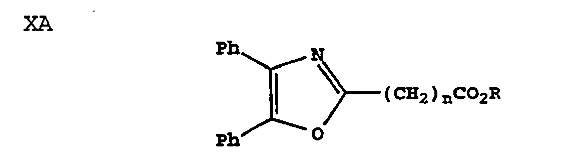 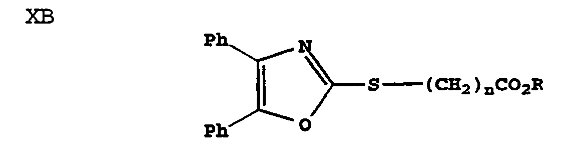 (wherein n is 7-9 and R is hydrogen or lower alkyl; or when R is hydrogen, the alkali metal salt thereof), 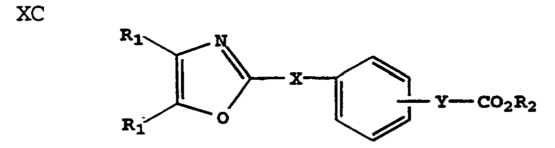 or 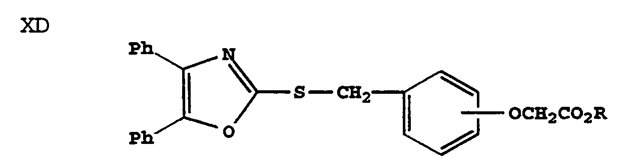 wherein
R₁ is phenyl or thienyl;
R₂ is hydrogen, lower alkyl or together with CO₂ is tetrazol-1-yl;
X is a divalent connecting group selected from the group consisting of CH₂CH₂, CH=CH, and CH₂O;
Y is a divalent connecting group attached to the 3-or 4-phenyl position selected from the group consisting of OCH₂, CH₂CH₂ and CH=CH,
or when R₂ is hydrogen, an alkali metal salt thereof.

Preferred are the preferred compounds as set out in the above Meanwell et al patent as illustrated in the working Examples thereof.

In another embodiment of the invention, compounds which have activity as aP2 inhibitors suitable for use herein are disclosed in PCT application WO 92/04334 which are substituted 4,5-diaryl heterocycles having the formula 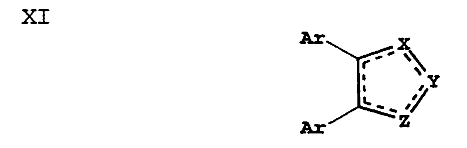 in which
each group Ar is the same or different and is optionally substituted phenyl or optionally substituted heteroaryl;
X is nitrogen or CR¹;
Y is nitrogen, N(CH₂)ₙA or C(CH₂)ₙA;
Z is nitrogen, oxygen or N(CH₂)ₙA, and the dotted line indicates the optional presence of a double bond so as to form a fully unsaturated heterocyclic ring;
R¹ is hydrogen, C₁₋₄alkyl, optionally substituted phenyl or optionally substituted heteroaryl;
n is 4 to 12; and
A is CO₂H or a group hydrolysable to CO₂H, 5-tetrazolyl, SO₃H, P(O) (OR)₂, P(O) (OH)₂, or P(O) (R) (OR) in which R is hydrogen or C₁₋₄alkyl, or a pharmaceutically acceptable salt thereof.

Preferred are preferred compounds of WO 92/04334.

In yet another embodiment of the invention, compounds which have activity as aP2 inhibitors suitable for use herein are disclosed in French Patent 2156486 which have the structure 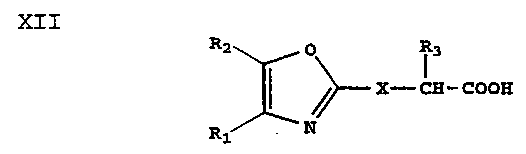 Where X is O or S;
R₁ is H, phenyl or phenyl substituted with F, Cl or Br or alkoxy,
R₂ is H, alkyl, phenyl or phenyl substituted with F, Cl or Br or alkoxy, and
R₃ is H or alkyl.

Preferred are those preferred compounds as set out in French Patent No. 2156486.

Most preferred oxazole compounds as aP2 inhibitors are the compounds 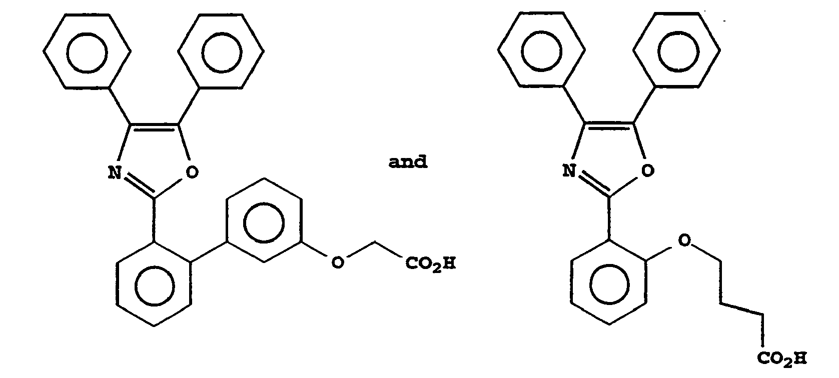 which may be prepared as disclosed in U.S. Patent No. 5,348,969 to Romine et al.

Another class of aP2 inhibitors suitable for use in the method of the invention include pyrimidine derivatives. Thus, U.S. Patent No. 5,599,770 to Kubota et al disclose compounds which have activity as aP2 inhibitors and thus suitable for use herein include 2-benzyloxypyrimidine derivatives having the following structure 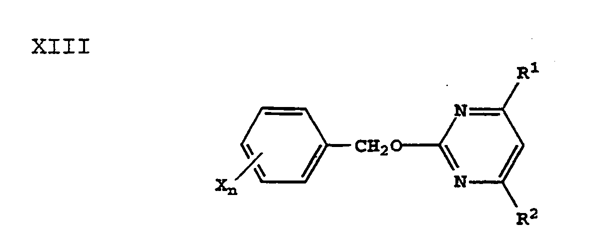 wherein
R¹ and R² are each independently H, a halogen, hydroxyl, C₁-C₄ alkyl, C₁-C₄ haloalkyl, C₃-C₅ alkenyl, C₃-C₅ alkynyl, C₁-C₄ alkoxy, C₁-C₄ haloalkoxy, C₃-C₅ alkenyloxy, C₃-C₅ alkynyloxy, C₁-C₄ alkylthio, or phenyl, with the proviso that at least one of R¹ and R² must be hydroxyl;
n is an integer of 0 to 5; and
each X which may be identical or different if n is greater than 1, is a halogen, C₁-C₄ alkyl, C₁-C₄ haloalkyl, C₁-C₄ alkoxy, C₁-C₄ alkylthio, C₇-C₉ aralkyloxy, phenyl, hydroxymethyl, hydroxycarbonyl, C₁-C₄ alkoxycarbonyl, or nitro.

Preferred are the compounds in which either R¹ or R² is hydroxyl and the other R¹ or R² is C₁-C₄ alkyl and X is halogen.

In another embodiment of the method of the invention, compounds which have activity as aP2 inhibitors suitable for use herein are disclosed in A. Mai et al "Dihydro(alkylthio)-(naphthylmethyl)oxopyrimidines: Novel Non-Nucleoside Reverse Transcriptase Inhibitors of the S-DABO Series", J. Med. Chem., 1997, 40, 1447-1454 which have the structures 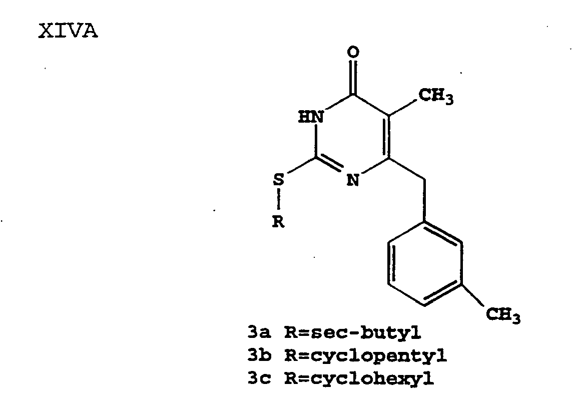 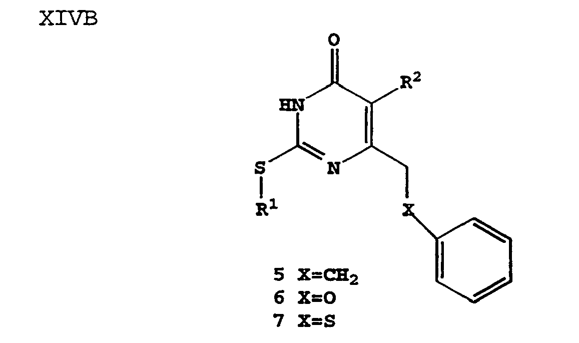 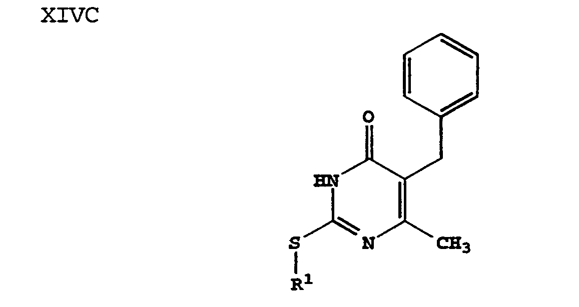 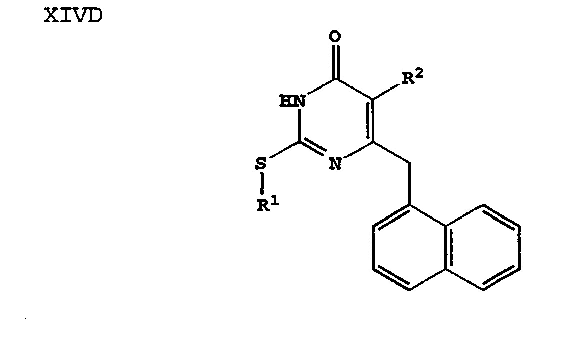 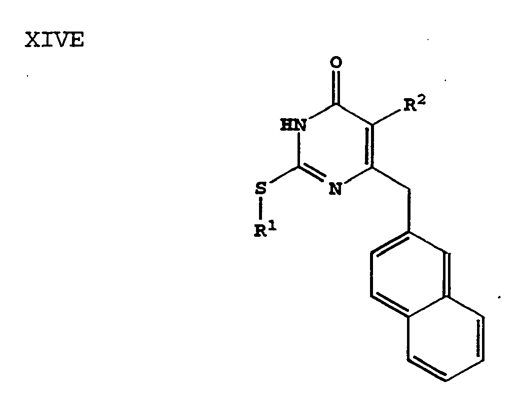 R¹ = sec-butyl, cyclopentyl, cyclohexyl;
R² = H, CH₃. The structures XIVA-XIVE are depicted in their keto form. However, it will be apparent to one skilled in the art that they may also exist in their enol form to give structures of the type

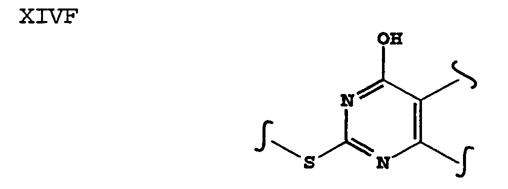

In yet another embodiment of the method of the invention, compounds which have activity as aP2 inhibitors suitable for use herein are disclosed in PCT appliction WO 96/35678 which are α-substituted pyrimidirie-thioalkyl and alkylether compounds which have the structure 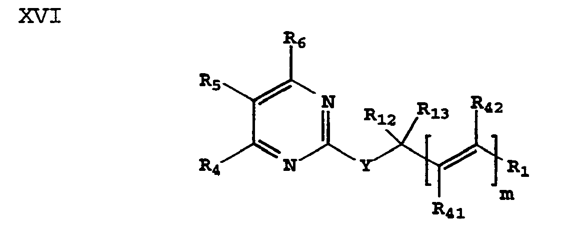 where m is 0 or 1;
R¹ is selected from -CO₂R₅₃, -CONR₅₄R₅₅, 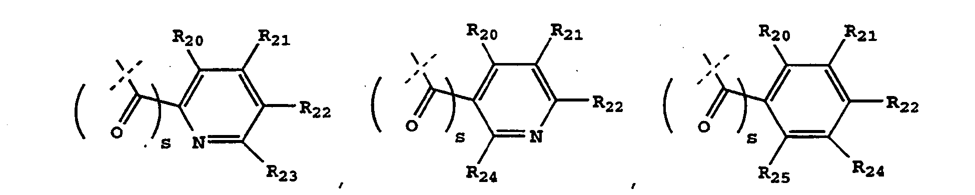 where s is 0 or 1, and R₂₀, R₂₁, R₂₂, R₂₃, R₂₄, and R₂₅ are the same or different and are selected from -H, C₁-C₆ alkyl, C₁-C₆ alkenyl, C₁-C₆ alkoxy, C₁-C₆ alkylthio, C₃-C₈ cycloalkyl, -CF₃, -NO₂, -halo, -OH, -CN, phenyl, phenylthio, -styryl, -CO₂ (R₃₁), -CON (R₃₁) (R₃₂), -CO(R₃₁), - (CH₂)ₙ-N(R₃₁) (R₃₂), -C(OH) (R₃₁(R₃₃), -(CH₂)ₙN(R₃₁) (CO(R₃₃)), (CH₂) ₙN (R₃₁) (SO₂ (R₃₃)), or where R₂₀ and R₂₁, or R₂₁ and R₂₂, or R₂₂ and R₂₃ are taken together to form a five or six-membered saturated or unsaturated ring containing 0 or 1 oxygen, nitrogen or sulfur, where the unsaturated ring may be optionally substituted with 1, 2 or 3, C₁-C₆ alkyl, C₁-C₆ alkoxy, -OH, -CH₂OH, -(CH₂)ₙ-N(R₃₁)(R₃₂), -C₃-C₈ cycloalkyl, -CF₃, -halo, CO₂(R₃₁), -CON(R₃₁)(R₃₂), -CO(R₃₁), - (CH₂) ₙN (R₃₁) (CO (R₃₃)), -(CH₂) ₙN (R₃₁) (SO₂ (R₃₃)), -CN, -CH₂CF₃ or -CH(CF₃)₂, or phenyl and the saturated ring may be optionally substituted with 1, 2 or 3, -C₁-C₆ alkyl, -C₁-C₆ alkoxy, -OH, -CH₂OH or -(CH₂)ₙ-N(R₃₁)(R₃₂) or one oxo (=O);
where n is 0-3 and R₃₁, R₃₂ and R₃₃ are the same or different and are selected from
-H,
C₁-C₆ alkyl,
phenyl optionally substituted with 1, 2 or 3 -halo, C₁-C₆ alkyl, C₁-C₆ alkoxy, -CF₃, -OH or -CN,
or where R₃₁ and R₃₂ taken together with the attached nitrogen to form a ring selected from -pyrrolidinyl, - piperidinyl, -4-morpholinyl, -4-thiomorpholinyl, -4-piperazinyl, -4-(1-C₁-C₆alkyl)piperazinyl, or a member selected from:
1-cyclohexenyl, 2-pyrimidinyl, 4-pyrimidinyl, 5-pyrimidinyl, 2-imidazolyl, 4-imidazolyl, 2-benzothiazolyl, 2-benzoxazolyl, 2-benzimidazolyl, 2-oxazolyl, 4-oxazolyl, 2-thiazolyl, 3-isoxazolyl, 5-isoxazolyl, 5-methyl-3-isoxazolyl, 5-phenyl-3-isoxazolyl, 4-thiazolyl, 3-methyl-2-pyrazinyl, 5-methyl-2-pyrazinyl, 6-methyl-2-pyrazinyl, 5-chloro-2-thienyl, 3-furyl, benzofuran-2-yl, benzothien-2-yl, 2H-1-benzopyran-3-yl, 2,3-dihydrobenzopyran-5-yl, 1-methylimidazol-2-yl, quinoxalin-2-yl, piperon-5-yl, 4,7-dichlorobenzoxazol-2-yl, 4,6-dimethylpyrimidin-2-yl, 4-methylpyrimidin-2-yl, 2,4-dimethylpyrimidin-6-yl, 2-methylpyrimidin-4-yl, 4-methylpyrimidin-6-yl, 6-chloropiperon-5-yl, 5-chloroimidazol[1,2-a]pyridin-2-yl, 1-H-inden-3-yl, 1-H-2-methyl-inden-2-yl, 3,4-dihydronaphth-1-yl, S-4-isopropenylcyclohexen-1-yl or 4-dihydronaphth-2-yl;
where R₅₃ is selected from -H, C₁-C₆alkyl, C₃-C₆cycloalkyl, phenyl (optionally substituted with 1, 2, or 3 -halo, C₁-C₆ alkyl, C₁-C₆ alkoxy, -CF₃, -OH, -CN), or a five or six-membered unsaturated ring containing 0 or 1 oxygen, nitrogen or sulfur, where the unsaturated ring may be optionally substituted with -H, C₁-C₆ alkyl, C₁-C₆ alkoxy, -OH, -CH₂OH, or -(CH₂)ₙ-N(R₃₁) (R₃₂) ;
where R₅₄ and R₅₅ being the same or different are selected from -H, C₁-C₆ alkyl, allyl, or phenyl (optionally substituted with 1, 2 or 3 -halo, C₁-C₆ alkyl, C₁-C₆ alkoxy or -CF₃), or taken together with the attached nitrogen to form a ring selected from -pyrrolidinyl, -piperidinyl, -4-morpholinyl, -4-thiomorpholinyl, -4-piperazinyl, -4-(l-C₁-C₆alkyl)piperazinyl;
R₄₁ and R₄₂, being the same or different, are selected from -H and C₁-C₄ alkyl;
R₁₂ is selected from -H, C₁-C₆ alkyl, -C₃-C₆ cycloalkyl, -CN, -C(O)NH₂, -C(O)N(C₁-C₆alkyl) (C₁-C₆alkyl), CO₂H, -CO₂(C₁-C₆alkyl), -CH₂OH, -CH₂NH₂ or -CF₃;
R₁₃ is selected from -H, C₁-C₆ alkyl or -CF₃;
Y is selected from -S-, -S(O)-, -S(O)₂, or -O-;
R₄ is -OH;
R₅ is selected from -H, -C₂H₄OH, -C₂H₄-O-TBDMS, halo, -C₃-C₆ cycloalkyl, C₁-C₃ alkoxy, -CH₂CH₂Cl or C₁-C₄ alkyl, with the proviso that R₅ is not isobutyl;
or, when R₆ is hydroxyl, R₄ and R₅ are taken together to form a five or six-memebered saturated or unsaturated ring which together with the pyrimidine ring form the group consisting of 7H-pyrrolo[2,3-d]pyrimidine, 5,6-dihydro-7H-pyrrolo[2,3-d]pyrimidine, furo[2,3-d]pyrimidine, 5,6-dihydro-furo[2,3-d]pyrimidine, thieno[2,3-d]pyrimidine, 5,6-dihydro-thieno[2,3-d]pyrimidine, 1H-pyrazolo[3,4-d]pyrimidine, 1H-purine, pyrimido[4,5-d]pyrimidine, pteridine, pyrido[2,3-d]pyrimidine, or quinazoline, where the unsaturated ring may be optionally substituted with 1, 2 or 3, C₁-C₆ alkyl C₁-C₆ alkoxy, -OH, -CH₂OH, or -(CH₂)ₙ-N (R₃₁) (R₃₂), -C₃-C₈ cycloalkyl, -CF₃, -halo, -CO₂(R₃₁), - CON(R₃₁) (R₃₂), -CO(R₃₁), -(CH₂)ₙN(R₃₁) (CO(R₃₃)), - (CH₂)ₙN(R₃₁)(SO₂(R₃₃)), and the saturated ring may be optionally substituted with 1, 2 or 3, -C₁-C₆ alkyl, C₁-C₆ alkoxy, -OH, -CH₂OH, or -(CH₂)ₙ-N(R₃₁)(R₃₂) or one oxo (=O); and
R₆ is selected from -H, -OH, halo, -CN, -CF₃, - CO₂ (R₆₁), -C (O) R₆₁ or -C (O) N(R₆₁) (R₆₂) where R₆₁ and R₆₂ are the same or different and are selected from
-H,
C₁-C₆ alkyl,
phenyl optionally substituted with 1, 2 or 3 -halo, C₁-C₆ alkyl, C₁-C₆ alkoxy, -CF₃, -OH, -CN,
or where R₆₁ and R₆₂ taken together with the attached nitrogen to form a ring selected from -pyrrolidinyl, - piperidinyl, -4-morpholinyl, -4-thiomorpholinyl, -4-piperazinyl, or -4-(C₁-C₆ alkyl)piperazinyl; or
pharmaceutically acceptable salts, hydrates, N-oxides and solvates thereof.

A preferred embodiment is pyrimidine-thioalkyl and alkylether, where
R₄ is -OH; and
R₆ is selected from -H, halo, -CN, -CF₃, -CO₂(R₁₆), - C (O) R₆₁ or -C (O) N (R₆₁) (R₆₂), preferably CF₃.

A preferred embodiment are compounds of Formula XVI where s is 0 or 1, and Y is -S- or O; more preferably Y is -S-.

Preferred are pyrimidine derivatives of the structures 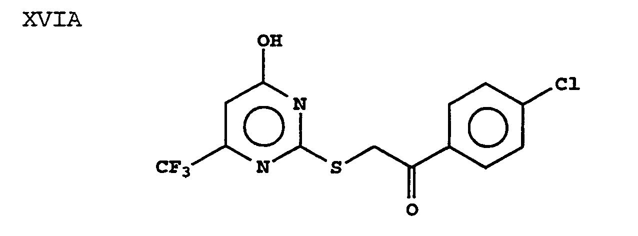 and 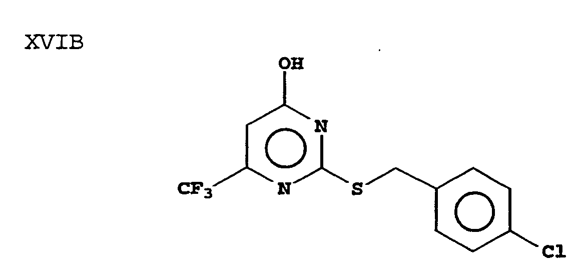 which may be prepared as disclosed in WO 96/35678.

Another embodiment of the method of the invention includes use of aP2 inhibitors which are pyridazinone derivatives. French Patent No. 2,647,676 discloses compounds which have activity as aP2 inhibitors and thus suitable for use herein which have the structures 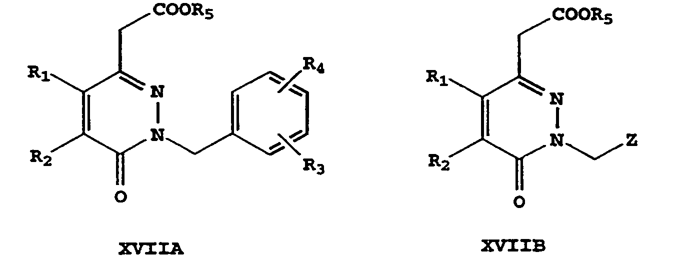 where R₁ and R₂ are H, alkyl, aryl or arylalkyl, where the alkyl can include as substituents halogen, CF₃, CH₃O, CH₃S, NO₂, or R₁ and R₂ with the carbons to which they are attached can form methylenedioxy, or
R₁ and R₂ can form a C₃-C₇ non-aromatic ring, or a heterocycle which can be pyridine, pyrazine, pyrimidine, pyridazine, indol, or pyrazole, or an oxygen containing heterocycle which can be pyran or furan, or a sulfur containing heterocycle which can be thiopyran, or thiophene; the heterocycles being optionally substituted with halogen or alkyl,
R₃ and R₄ are H, alkyl, halogen, CF₃, CH₃O, CH₃S or NO₂ or R₃ and R₄ with the carbons to which they are attached can form a methylenedioxy group,
R₅ is H, and
Z is a heterocycle which can be pyridine, thiazole, benzothiazole, benzimidazole or quinoline, which Z group can optionally be substituted with halogen or alkyl.

The preferred pyridazinone derivative is 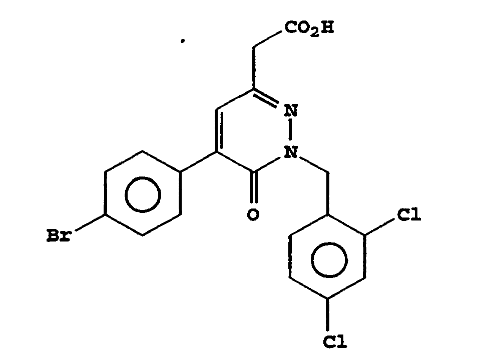 which may be prepared as disclosed in French Patent No. 2,647,676.

Preferred aP2 inhibitors for use herein will include an oxazole ring.

Unless otherwise indicated, the term "lower alkyl", "alkyl" or "alk" as employed herein alone or as part of another group includes both straight and branched chain hydrocarbons, containing 1 to 40 carbons, preferably 1 to 20 carbons, more preferably 1 to 12 carbons, in the normal chain, such as methyl, ethyl, propyl, isopropyl, butyl, t-butyl, isobutyl, pentyl, hexyl, isohexyl, heptyl, 4,4-dimethylpentyl, octyl, 2,2,4-trimethyl-pentyl, nonyl, decyl, undecyl, dodecyl, the various branched chain isomers thereof, and the like as well as such groups including 1 to 4 substituents such as halo, for example F, Br, Cl or I or CF₃, alkoxy, aryl, aryloxy, aryl(aryl) or diaryl, arylalkyl, arylalkyloxy, alkenyl, cycloalkyl, cycloalkylalkyl, cycloalkylalkyloxy, amino, hydroxy, acyl, heteroaryl, heteroaryloxy, heteroarylalkyl, heteroarylalkoxy, aryloxyalkyl, aryloxyaryl, alkylamido, alkanoylamino, arylcarbonylamino, nitro, cyano, thiol, haloalkyl, trihaloalkyl and/or alkylthio.

Unless otherwise indicated, the term "cycloalkyl" as employed herein alone or as part of another group includes saturated or partially unsaturated (containing 1 or 2 double bonds) cyclic hydrocarbon groups containing 1 to 3 rings, including monocyclicalkyl, bicyclicalkyl and tricyclicalkyl, containing a total of 3 to 20 carbons forming the rings, preferably 4 to 12 carbons, forming the ring and which may be fused to 1 or 2 aromatic rings as described for aryl, which include cyclopropyl, cyclobutyl, cyclopentyl, cyclohexyl, cycloheptyl, cyclooctyl, cyclodecyl and cyclododecyl, cyclohexenyl, 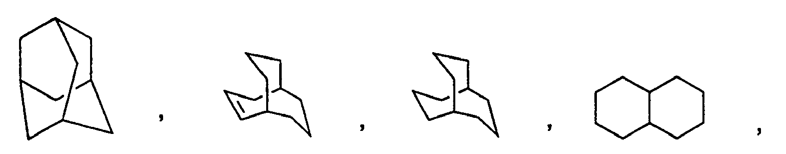 any of which groups may be optionally substituted with 1 to 4 substituents such as halogen, alkyl, alkoxy, hydroxy, aryl, aryloxy, arylalkyl, cycloalkyl, alkylamido, alkanoylamino, oxo, acyl, arylcarbonylamino, amino, nitro, cyano, thiol and/or alkylthio.

Unless otherwise indicated the term "aryl" or "Ar" as employed herein alone or as part of another group refers to monocyclic and bicyclic aromatic groups containing 6 to 10 carbons in the ring portion (such as phenyl or naphthyl) and may optionally include one to three additional rings fused to Ar (such as aryl, cycloalkyl, heteroaryl or cycloheteroalkyl rings) and may be optionally substituted through available carbon atoms with 1, 2, 3 or 4 groups selected from hydrogen, halo, haloalkyl, alkyl, haloalkyl, alkoxy, haloalkoxy, alkenyl, trifluoromethyl, trifluoromethoxy, alkynyl, cycloalkylalkyl, cycloheteroalkyl, cycloheteroalkylalkyl, aryl, heteroaryl, arylalkyl, aryloxy, aryloxyalkyl, arylalkoxy, arylthio, arylazo, heteroarylalkyl, heteroarylalkenyl, heteroarylheteroaryl, heteroaryloxy, hydroxy, nitro, cyano, amino, substituted amino wherein the amino includes 1 or 2 substituents (which are alkyl, aryl or any of the other aryl compounds mentioned in the definitions), thiol, alkylthio, arylthio, heteroarylthio, arylthioalkyl, alkoxyarylthio, alkylcarbonyl, arylcarbonyl, alkylaminocarbonyl, arylaminocarbonyl, alkoxycarbonyl, aminocarbonyl, alkylcarbonyloxy, arylcarbonyloxy, alkylcarbonylamino, arylcarbonylamino, arylsulfinyl, arylsulfinylalkyl, arylsulfonylamino or arylsulfonaminocarbonyl.

Unless otherwise indicated the term "aralkyl", "aryl-alkyl" or "aryllower alkyl" as used herein alone or as part of another group refers to alkyl groups as discussed above having an aryl substituent, such as benzyl or phenethyl, or naphthylpropyl, or an aryl as defined above.

Unless otherwise indicated, the term "cycloheteroalkyl" as used herein alone or as part of another group refers to a 5-, 6- or 7-membered saturated or partially unsaturated ring which includes 1 to 2 hetero atoms such as nitrogen, oxygen and/or sulfur, linked through a carbon atom or a heteroatom, where possible, optionally via the linker (CH₂)ₚ (where p is 1, 2 or 3), such as 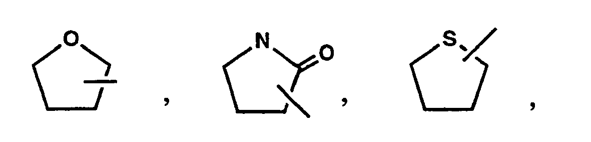 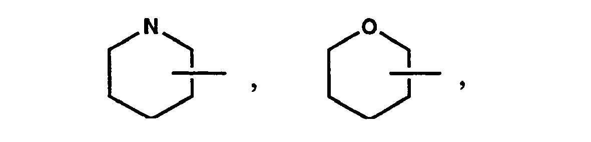 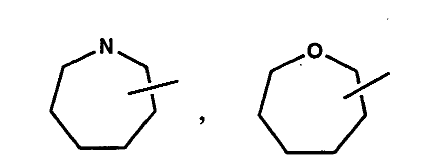 and the like. The above groups may include 1 to 3 substituents such as any of the substituents for alkyl or aryl as defined above. In addition, any of the above rings can be fused to 1 or 2 cycloalkyl, aryl, heteroaryl or cycloheteroalkyl rings.

Unless otherwise indicated, the term "heteroaryl" (also referred to as heteroaryl) as used herein alone or as part of another group refers to a 5- or 6-membered aromatic ring which includes 1, 2, 3 or 4 hetero atoms such as nitrogen, oxygen or sulfur, and such rings fused to an aryl, cycloalkyl, heteroaryl or cycloheteroalkyl ring (e.g. benzothiophenyl, indolyl), linked through a carbon atom or a heteroatom, where possible, optionally via the linker (CH₂)ₚ (which is defined above), such as 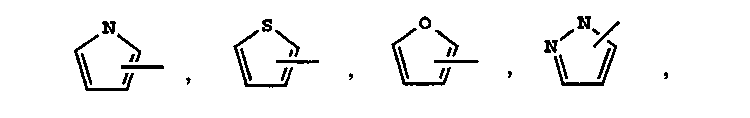 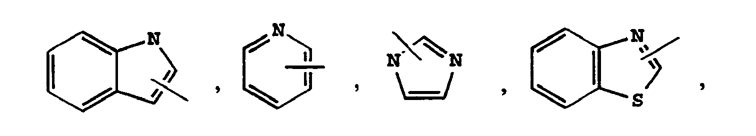 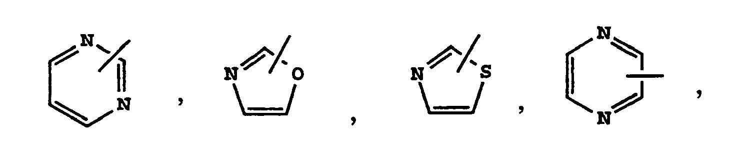 and the like.

The heteroaryl groups including the above groups may optionally include 1 to 4 substituents such as any of the substituents listed for aryl. In addition, any of the above rings can be fused to a cycloalkyl, aryl, heteroaryl or cycloheteroalkyl ring.

The term "prodrug esters" as employed herein includes prodrug esters which are known in the art for both phosphorus and carboxylic acids such as similar carboxylic acid esters such as methyl, ethyl benzyl and the like. Other examples include the following groups: (1-alkanoyloxy)alkyl such as, 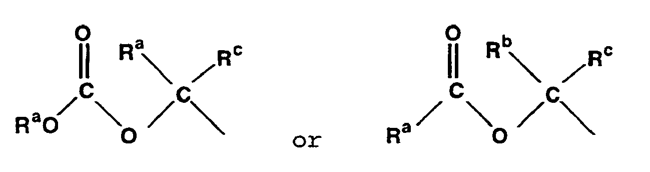 wherein R^{a}, R^{b} and R^{c} are H, alkyl, aryl or aryl-alkyl; however R^{a}O cannot be HO. Examples of such prodrug esters include 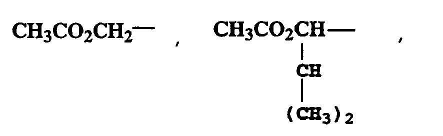 t-C₄H₉CO₂CH₂-, or 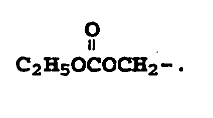 Other examples of suitable prodrug esters include 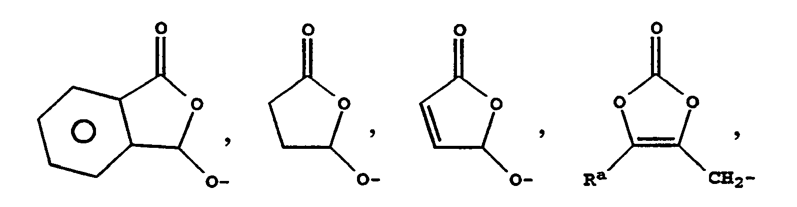 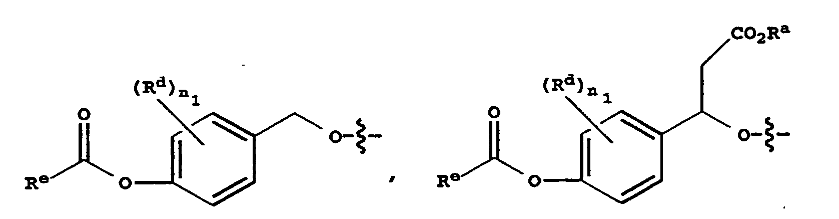 wherein R^{a} can be H, alkyl (such as methyl or t-butyl), arylalkyl (such as benzyl) or aryl (such as phenyl); R^{d} is H, alkyl, halogen or alkoxy, R^{e} is alkyl, aryl, arylalkyl or alkoxyl, and n₁ is 0, 1 or 2; or

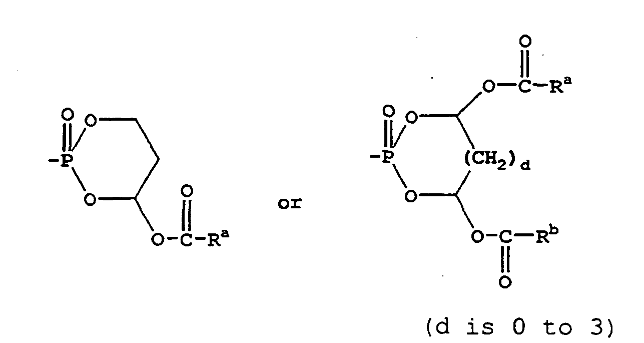

Where the aP2 inhibitor is in acid form it may form a pharmaceutically acceptable salt such as alkali metal salts such as lithium, sodium or potassium, alkaline earth metal salts such as calcium or magnesium as well as zinc or aluminum and other cations such as ammonium, choline, diethanolamine, ethylenediamine, t-butylamine, t-octylamine, dehydroabietylamine.

Where desired, the aP2 inhibitor may be used in combination with another antidiabetic agent (also referred to herein as "another antihyperglycemic agent") which may be administered orally in the same dosage form in accordance with the invention, a separate oral dosage form or by injection.

The other antidiabetic agent may be a biguanide, a sulfonyl urea, a glucosidase inhibitor, a thiazolidinedione, an insulin sensitizer, a glucagon-like peptide-1 (GLP-1), insulin or a PPAR α/γ dual agonist and/or a meglitinide.

It is believed that the use of the aP2 inhibitor in combination with another antidiabetic agent produces antihyperglycemic results greater than that possible from each of these medicaments alone and greater than the combined additive anti-hyperglycemic effects produced by these medicaments.

The other antidiabetic agent may be an oral antihyperglycemic agent preferably a biguanide such as metformin or phenformin or salts thereof.

Where the other antidiabetic agent is a biguanide, the aP2 inhibitor will be employed in a weight ratio to biguanide within the range from about 0.01:1 to about 100:1, preferably from about 0.5:1 to about 2:1.

The other antidiabetic agent may also preferably be a sulfonyl urea such as glyburide (also known as glibenclamide), glimepiride (disclosed in U.S. Patent No. 4,379,785), glipizide, gliclazide or chlorpropamide, other known sulfonylureas or other antihyperglycemic agents which act on the ATP-dependent channel of the β-cells, with glyburide being preferred.

The aP2 inhibitor will be employed in a weight ratio to the sulfonyl urea in the range from about 0.01:1 to about 100:1, preferably from about 0.2:1 to about 10:1.

The oral antidiabetic agent may also be a glucosidase inhibitor such as acarbose (disclosed in U.S. Patent No. 4,904,769) or miglitol (disclosed in U.S. Patent No. 4,639,436), which may be administered in a separate oral dosage form.

The aP2 inhibitor will be employed in a weight ratio to the glucosidase inhibitor within the range from about 0.01:1 to about 100:1, preferably from about 0.5:1 to about 50:1.

The aP2 inhibitor may be employed in combination with a thiazolidinedione oral antidiabetic agent or other insulin sensitizers (which has an insulin sensitivity effect in NIDDM patients) such as troglitazone (Warner-Labert's Rezulin^{®}, disclosed in U.S. Patent No. 4,572,912), rosiglitazone (SKB), pioglitazone (Takeda), Mitsubishi's MCC-555 (disclosed in U.S. Patent No. 5,594,016), Glaxo-Welcome's GL-262570, englitazone (CP-68722, Pfizer) or darglitazone (CP-86325, Pfizer).

The aP2 inhibitor will be employed in a weight ratio to the thiazolidinedione in an amount within the range from about 0.01:1 to about 100:1, preferably from about 0.5:1 to about 5:1.

The sulfonyl urea and thiazolidinedione in amounts of less than about 150 mg oral antidiabetic agent may be incorporated in a single tablet with the aP2 inhibitor.

The aP2 inhibitor may also be employed in combination with a non-oral antihyperglycemic agent such as insulin or with glucagon-like peptide-1 (GLP-1) such as GLP-1(1-36) amide, GLP-1(7-36) amide, GLP-1(7-37) (as disclosed in U.S. Patent No. 5,614,492 to Habener, which may be administered via injection, or by transdermal or buccal devices.

Where present, metformin, the sulfonyl ureas, such as glyburide, glimepiride, glipyride, glipizide, chlorpropamide and gliclazide and the glucosidase inhibitors acarbose or miglitol or insulin may be employed in formulations as described above and in amounts and dosing as indicated in the Physician's Desk Reference.

Where present, metformin or salt thereof may be employed in amounts within the range from about 500 to about 2000 mg per day which may be administered in single or divided doses one to four times daily.

Where present, the thiazolidinedione antidiabetic agent may be employed in amounts within the range from about 0.01 to about 2000 mg/day which may be administered in single or divided doses one to four times per day.

Where present, insulin may be employed in formulations, amounts and dosing as indicated by the Physician's Desk Reference.

Where present, GLP-1 peptides may be administered in oral buccal formulations, by nasal administration or parenterally as described in U.S. Patent Nos. 5,346,701 (TheraTech), 5,614,492 and 5,631,224 which are incorporated herein by reference.

The aP2 inhibitor may be employed in combination with another antidiabetic agent which may be a PPAR α/γ dual agonist such as an N-benzyldioxothiazolidylbenzamide derivative such as disclosed in WO 96/38428 such as 5-(2,4-dioxothiazolidin-5-ylmethyl)-2-methoxy-N-[4-(trifluoromethyl)benzyl]benzamide (KRP-297), WO 98/05531 (Ligand Pharmaceuticals, Inc.) which discloses 2-(4-[2,4-difluorophenyl]-1-heptylureido)ethyl]phenoxy)-2-methylbutyric acid, and WO 97/25042 and WO96/04260 (SKB) which disclose benzoxazole and pyridine derivatives of the structure 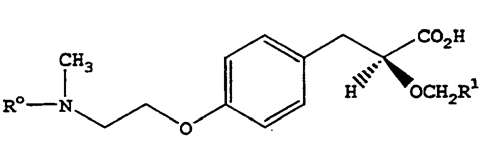 or a pharmaceutically acceptable salt thereof, and/or a pharmaceutically acceptable solvate thereof, wherein R^{o} represents 2-benzoxazolyl or 2-pyridyl and R¹ represents CH₂OCH₃ or CF₃, such as (S)-3-[4-[2-[N-(2-benzoxazolyl)-N-methylamino)ethoxy]phenyl]-2-(2-methoxy-ethoxy)propanoic acid; or (S)-3-[4-[2-[N-(2-benzoxazolyl)-N-methylamino]-ethoxy]phenyl]-2-(2,2,2-trifluoroethoxy)propanoic acid; or a pharmaceutically acceptable salt thereof, and/or a pharmaceutically acceptable solvate thereof. Dosages employed are as set out in the above references.

The aP2 inhibitor will be employed in a weight ratio to the PPAR α/γ dual agonist within the range from about 0.01:1 to about 100:1, preferably from about 0.5:1 to about 5:1.

Where the aP2 inhibitor is employed in combination with the PPAR α/γ dual agonist, the combination may be employed in an oral dosage form such as a tablet or capsule as will be apparent to one skilled in the art.

Where present, the meglitinide, for example, repaglinide (Prandin^{®}, Novartis) or nataglinide (Starlix^{®}, Novartis) may be employed in formulations, amounts and dosing as indicated in the Physician's Desk Reference.

The aP2 inhibitor will be employed in a weight ratio to the meglitinide within the range from about 0.01:1 to about 100:1, preferably from about 0.5:1 to about 50:1.

In carrying our the method of the invention, a pharmaceutical composition will be employed containing at least one aP2 inhibitor with or without another antidiabetic agent in association with a pharmaceutical vehicle or diluent. The pharmaceutical composition can be formulated employing conventional solid or liquid vehicles or diluents and pharmaceutical additives of a type appropriate to the mode of desired administration. The compounds can be administered to mammalian species including humans, monkeys, dogs, etc. by an oral route, for example, in the form of tablets, capsules, granules or powders, or they can be administered by a parenteral route in the form of injectable preparations. The dose for adults is preferably between 50 and 2,000 mg per day, which can be administered in a single dose or in the form of individual doses from 1-4 times per day.

A typical capsule for oral administration contains aP2 inhibitor (250 mg), lactose (75 mg) and magnesium stearate (15 mg). The mixture is passed through a 60 mesh sieve and packed into a No. 1 gelatin capsule.

A typical injectable preparation is produced by aseptically placing 250 mg of aP2 inhibitor into a vial, aseptically freeze-drying and sealing. For use, the contents of the vial are mixed with 2 mL of physiological saline, to produce an injectable preparation.

Compounds sufficiently satisfying the structural criteria described above may be determined by use of an in vitro assay system which measures the potentiation of inhibition of aP2 by displacement of a fluorescent substrate from aP2 by the inhibitor. Inhibition constants (Ki values) for the inhibitors may be determined by the method described below:
**Production of purified recombinant human aP2 protein.** Recombinant human aP2 protein is produced by standard recombinant DNA technology. In the typical case, aP2 is produced by heterologous expression in E. coli strain BL21(D53) transformed with pETlla vector containing the full length human aP2 cDNA (Baxa, C.A., Sha, R.S., Buelt, M.K., Smith, A.J., Matarese, V., Chinander, L.L., Boundy, K.L., and Bernlohr, D.A. (1989). Human adipocyte lipid-binding protein: purification of the protein and cloning of its complementary DNA. Biochemistry 28: 8683-8690 and Xu, Z., Buelt, M.K., Banaszak, L.J., and Bernlohr, D.A. (1991). Expression, purification and crystallization of the adipocyte lipid binding protein. J. Biol. Chem. 266: 14367-14370). Purification of aP2 from E. coli is conducted as described by Xu, yielding essentially homogeneous aP2 protein with molecular weight -14600 daltons and free of endogenous fatty acids. The purified aP2 is capable of binding up to one mole of free fatty acid per mole protein. The binding and structural properties of recombinant aP2 protein were previously shown to be identical to aP2 protein isolated from adipose tissue.
**In vitro assay of aP2 inhibitors.** Inhibitors of aP2 are evaluated in a homogeneous fluorescent-based competition assay using recombinant aP2 protein and 1,8-anilino-naphthalene-sulfonic acid (1,8-ANS) as assay substrate. This competition assay was adapted from generalized procedures described previously (Kane, C.D. and Bernlohr, D.A. (1996). A simple assay for intracellular lipid-binding proteins using displacement of 1-anilino-8-sulfonic acid. (1996) Anal. Biochem. 233: 197-204 and Kurian E., Kirk, W.R. and Prendergast, F.G. (1996) Affinity of fatty acid for r-rat intestinal fatty acid binding protein. Biochemistry, 35, 3865-3874). The method relies on the increase in fluorescence quantum yield of 1,8-ANS upon binding to the fatty acid binding site of aP2. The assay is run using appropriate concentrations of inhibitor, 1,8-ANS, and aP2 protein, in order to calculate the inhibitor binding constant (Ki) for compounds being evaluated. The Ki calculation was based on the procedure previously described for calculation of dissociation constants described by Kurian. Lower Ki values indicate higher affinities of compounds binding to aP2.

In the assay as conducted for the inhibitors described herein, a series of aliquots of aP2 (5 µM) in solution in 10 mM potassium phosphate buffer (pH 7.0) are mixed with an equimolar concentration of test compound, followed by the addition of a series of increasing concentrations of 1,8-ANS (from 0 to 5 µM). The assay typically is conducted in 96-well plate format with reagents added using robotic instrumentation (Packard Multiprobe 104). The fluorescence value for each test is determined using a Cytofluor-4000 multi-well fluorescence plate reader (Perceptive Biosystems) using excitation wavelength 360 nm and emission wavelength 460 nm, or using other suitable spectrofluorometer. In preparation for the assay, test compounds are initially prepared at 10 mM in dimethylsulfoxide. All subsequent dilutions and assay additions are made in 10 mM potassium phosphate buffer, pH 7.0.

X-ray crystallography of the inhibitor-aP2 complex can be performed by one skilled in the art using contemporary biophysical methodologies and commercial instrumentation. Such crystallographic data can be used to conclusively determine if a compound used in the present invention has embodied the structural requirement necessary for inhibition of aP2. An example of such an X-ray crystallographic determination is presented below:
Crystals of aP2 complexed with the inhibitors were typically grown by the hanging drop method. aP2, at 8.3 mg/ml, was pre-equilibrated with 1-5 mM of the inhibitor in 0.1 M Tris-HCl pH 8.0, 1% w/v DMSO for four hours. 2 µl drops containing equilibrated protein and reservoir solution at a 1:1 ratio were suspended on plastic cover slips and equilibrated against a 1 ml reservoir containing 2.6-3.0 M ammonium sulfate in 0.1 M Tris-HCl pH 8.0. Crystals typically appeared in 2-3 days and reached maximum size within 2 weeks. Data was typically collected on a single flash-frozen crystal (Oxford Cryosystems) using a Rigaku rotating anode and an R-axis II image plate detector of a Bruker multiwire area detector. Diffraction from aP2 crystals was excellent. Diffraction was consistently observed to better than 2.0 Å resolution often to beyond 1.5 Å resolution. Data was processed either with DENZO/SCALEPACK (R-axis II data), or Xengen (Bruker data). XPLOR was used for structure refinement and model building was done using the molecular modeling package CHAIN. After a single round of refinement, examination of the Fₒ-F_{c} map typically allowed facile building of the inhibitor into aP2 binding cavity. Iterative fitting and refinement were continued until improvement was no longer seen in the electron density map or R-free.

Referring to the accompanying Figure which is a computer generated image of a partial X-ray structure of compound XVIA bound to human aP2, the ball and stick figure in light gray is compound XVIA. The Arg106, Arg126, and Tyr128 residues are depicted as ball and stick figures in dark gray. The dark spheres represent a space filling view of the discrete binding pocket comprised of the residues Phe16, Tyr19, Met20, Val23, Val25, Alia33, Phe57, Thr74, Alia75, Asp76, Arg78. The 4-chlorophenyl substituent of compound XVIA is shown bound within this discrete pocket and the hydroxyl group is bound to the Arg-Tyr-Arg residues.

## Claims

1. Use of aP2 inhibitor for the preparation of a pharmaceutical composition for treating diabetes, insulin resistance, obesity, hyperglycemia, hyperinsulinemia, or elevated fatty acids, or glycerol, or hypertriglyceridemia.

2. The use as defined in Claim 1 wherein the aP2 inhibitor binds to the aP2 protein and inhibits its function and/or its ability to bind free fatty acids.

3. The use as defined in Claim 1 wherein the aP2 inhibitor contains a hydrogen bond donator or acceptor group and interacts directly or through an intervening water molecule either by ionic or hydrogen bonding interactions, with one, two, or three of the three amino, acid residues, designated as Arg 106, Arg 126 and Tyr 128 in human aP2 within the aP2 protein.

4. The use as defined in Claim 3 wherein the hydrogen bond donator or acceptor group is acid in nature.

5. The use as defined in Claim 3 where said aP2 inhibitor contains an additional substituent which binds to (in) and/or interacts with a discrete pocket within the aP2 protein defined roughly by the amino acid residues Phe 16, Tyr 19, Met 20, Val 23, Val 25, Ala 33, Phe 57, Thr 74, Ala 75, Asp 76, Arg 78 in human aP2.

6. The use as defined in Claim 5 wherein said additional substituent in said aP2 inhibitor is hydrophobic in nature.

7. The use as defined in Claim 5 in which the through space distance from the hydrogen bond donor/acceptor group and the additional substituent group in said aP2 inhibitors is within the distance of about 7 to about 15 Angstroms.

8. The use as defined in Claim 1 wherein Type II diabetes is treated.

9. The use as defined in Claim 1 wherein the aP2 inhibitor is employed in the form of a pharmaceutically acceptable salt thereof or a prodrug ester thereof.

10. The use as defined in Claim 1 wherein the aP2 inhibitor includes an oxazole or analogous ring, a pyrimidine derivative or a pyridazinone derivative.

11. The use as defined in Claim 10 wherein the aP2 inhibitor is a substituted benzoyl or biphenyl-2-oxazole-alkanoic acid derivative, an oxazole derivative, a 2-thio-4,5-diphenyloxazole S-derivative, a phenyl-heterocyclic oxazole derivative, a diaryloxazole derivative, a 4,5-diphenyloxazole derivative, an oxazole carboxylic acid derivative, a phenyloxazolyloxazole derivative, or a 2-(4,5-diaryl)-2-oxazolyl substituted phenoxyalkanoic acid derivative.

12. The use as defined in Claim 10 wherein the aP2 inhibitor is a 2-benzyloxypyrimidine derivative, a dihydro(alkylthio)(naphthylmethyl)oxypyrimidine derivative, a thiouracil derivative, or an α-substituted pyrimidine-thioalkyl or alkyl ether derivative.

13. The use as defined in Claim 10 wherein the aP2 inhibitor is a pyridazinone acetic acid derivative.

14. The use as defined in Claim 10 wherein the aP2 inhibitor is
(I)a substituted benzoylbenzene or biphenyl alkanoic acid derivative having the structure:
I A(CH₂) ₙO-B
wherein
A is a group having the formula 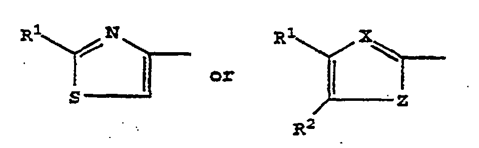 wherein
X is -N- or 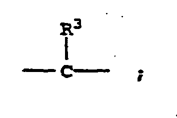 Z is 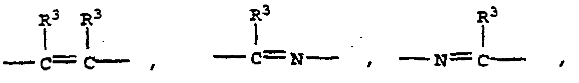 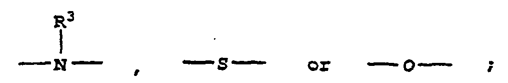 R¹ is hydrogen, lower alkyl or phenyl;
R² is hydrogen or lower alkyle; or
R¹ and R² taken together form a benzene ring, with the proviso that when X is -N-, Z is other than 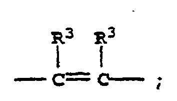 R³ is hydrogen or lower alkyl;
n is 1-2 ;
B is 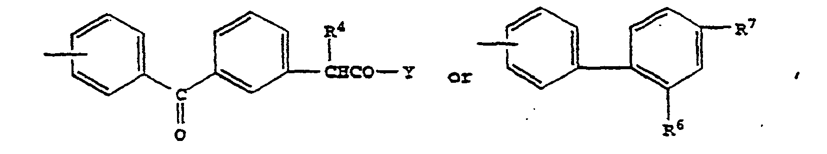 wherein
Y is OR⁵ or N(OH)R⁸;
R⁴ and R⁵ are each, independently, hydrogen or lower alkyl;
R⁶ is hydrogen, halo or nitro;
R⁷ is 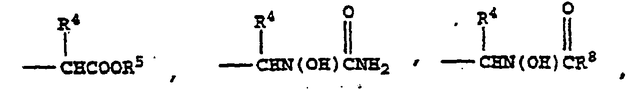 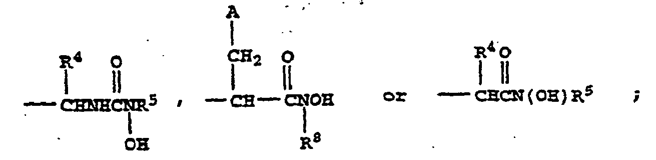 R⁸ is lower alkyl;
m is 0-3 ;
or a pharmacologically acceptable salts thereof;
(II) oxazole derivatives which have the structure 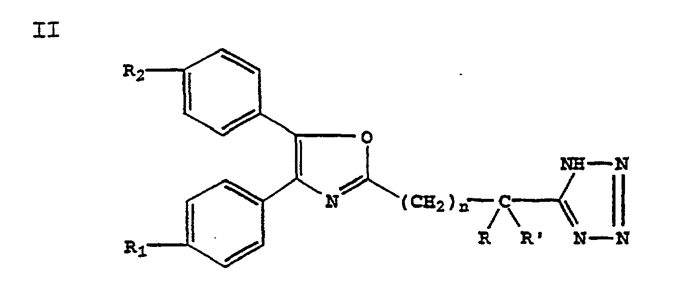 in which;
R and R' are identical or different and represent a hydrogen atom or an alkyl radical containing 1 or 2 carbon atoms,
R₁ and R₂ are identical or different and represent hydrogen or halogen atoms or alkyloxy radicals in which the alkyl portion contains 1 to 4 carbon atoms in a straight or branched chain, and
n equals 3 to 6, as well to their salts;
(III) 2-thiol-4,5-diphenyloxazole S-derivatives which have the structure 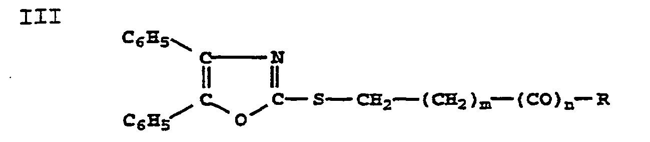 wherein m is 0, 1 or 2, n is 1 and R represents hydroxy, alkoxy or amino, and pharmaceutically acceptable addition salts thereof;
(IV) azole derivatives of the structure 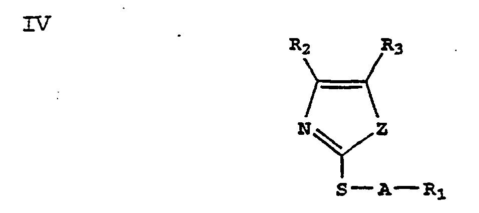 wherein R₁ is carboxyl, esterified carboxyl or other functionally modified carboxyl group; R₂ and R₃ each are aryl of up to 10 carbon atoms; A is CₙH₂ₙ in which n is an integer from 1 to 10, inclusive;:and Z is O or S, and physiologically acceptable salts thereof;
(V) phenyl-heterocyclic oxazole derivatives which have the structure 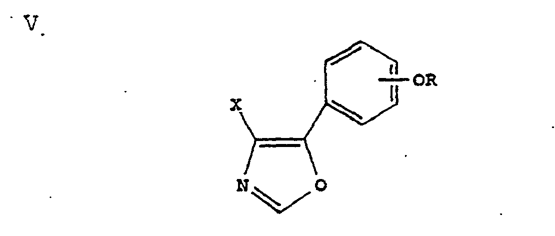 x is 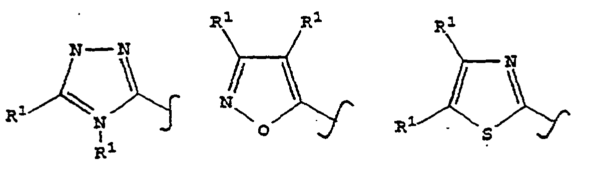 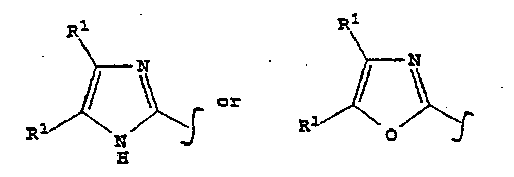 R is CH₂R²;
R¹ is Ph or Th;
R² is 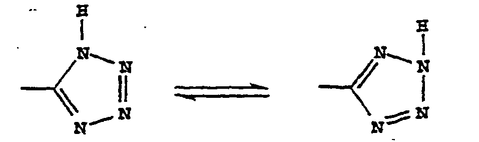 CO₂R³; and
R³ is H, or C₁-C₄ lower alkyl;
or pharmaceutically acceptable salt thereof;
(VI) diaryloxazole derivatives having the structure 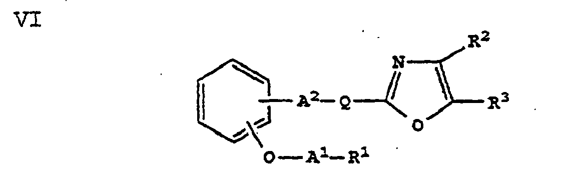 wherein R¹ is carboxy or protected carboxy,
R² is aryl,
R³ is aryl,
A¹ is lower alkylene,
A² is bond or lower alkylene and
-Q- is 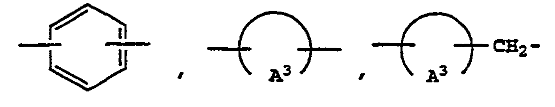 or 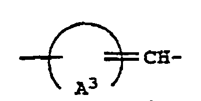 (in which 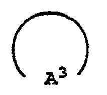 is cyclo (lower)alkane or
cycle(lower)alkene,
each of which may have suitable substituent(s));
(VII) 4,5-diphenyloxazole derivatives having the structure 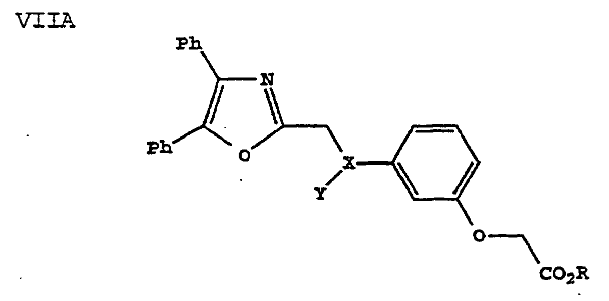 wherein
R is H or C₁-C₅ lower alkyl,
X is N or CH,
Y is H or CO₂R¹, or COR², provided that when X is CH, Y is not H,
R¹ is C₁-C₅ lower alkyl, or phenylmethyl, and
R² is C₁-C₅ alkyl; 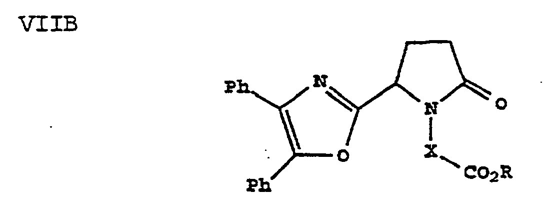 wherein
R is H or C₁-C₅ lower alkyl,
X is (CH₂)ₙ or para or meta substituted phenyl
wherein the substituent is OR²,
R² is C₁-C₅ alkyl, and
n is an integer of 4 to 8,
and pharmaceutically acceptable salts thereof;
(VIII) oxazole carboxylic acid derivatives having the structure 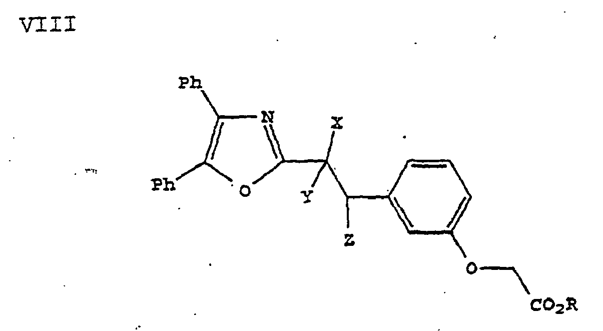 wherein
Y and Z are independently hydrogen or together form a bond;
X is CN, CO₂R¹- or CONR²R³;
R and R¹ are independently or together H, Na, or C₁-C₅ lower alkyl;
R² and R³ are independently or together H, or C₁-C₅ lower alkyl;
or alkali metal salt thereof;
(IX) phenyloxazolyloxazole derivatives having the structure 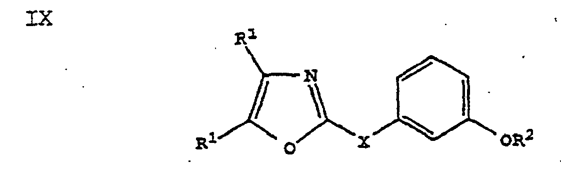 wherein
X is 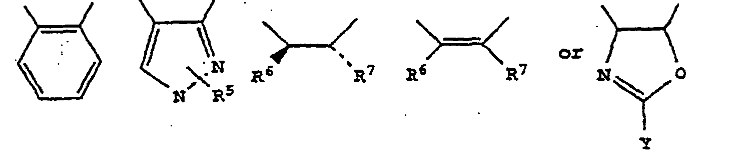 Y is CH₃, Ph, or OH, provided that when Y is OH, the compound exists in the keto-enol tautaumerism form 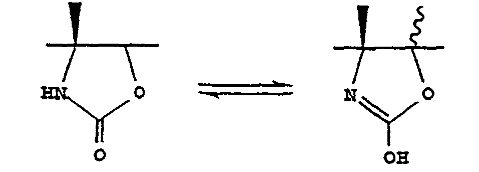 R¹ is Ph or Th;
R² is CH₂R³;
R³ is CO₂R⁴;
R⁴ is H or C₁-C₅ lower alkyl;
R⁵ is H or CH₃ ; R⁶ is OHCHN or H₂N; and
R⁷ is H or OH;
or pharmaceutically acceptable salt thereof;
(X) 2-(4,5-diaryl)-2-oxazolyl substituted phenoxyalkanoic acids and esters having the strucutre 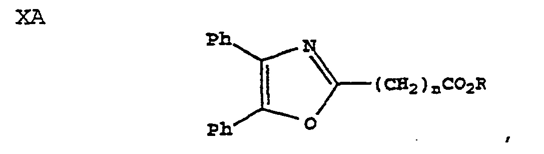 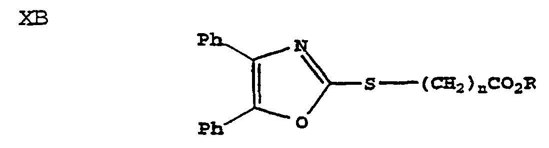 (wherein n is 7-9 and R is hydrogen or lower alkyl; or when R is hydrogen, the alkali metal salt thereof), 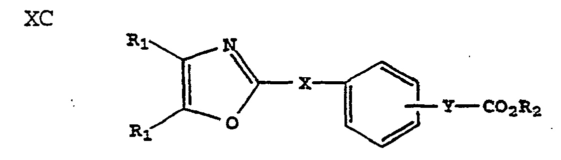 or 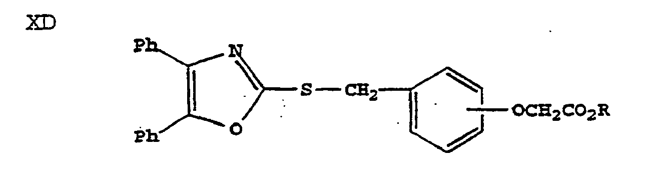 wherein
R₁ is phenyl or thienyl;
R₂ is hydrogen, lower alkyl or together with CO₂ is tetrazol-1-yl;
X is a divalent connecting group selected from the group consisting of CH₂CH₂, CH=CH, and CH₂O;
Y is a divalent connecting group attached to the 3-or 4-phenyl position selected.from the group consisting of OCH₂, CH₂CH₂ and CH=CH,
or when R₂ is hydrogen, an alkali metal salt thereof;
(XI) substituted 4,5-diaryl heterocycles having the formula 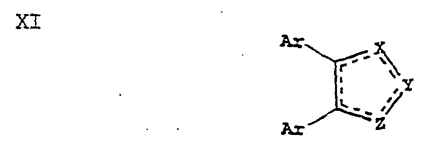 in which
each group Ar is the same or different and is optionally substituted phenyl or optionally substituted heteroaryl;
X is nitrogen or CR¹;
Y is nitrogen, N(CH₂)ₙA or C(CH₂)ₙA;
Z is nitrogen, oxygen or N(CH₂)ₙA, and the dotted line indicates the optional presence of a double bond so as to form a fully unsaturated heterocyclic ring;
R¹ is hydrogen, C₁₋₄alkyl, optionally substituted phenyl or optionally substituted heteroaryl;
n is 4 to 12; and
A is CO₂H or a group hydrolysable to CO₂H, 5-tetrazolyl, SO₃H, P(O) (OR)₂, P(O) (OH)₂, or P(O) (R) (OR) in which R is hydrogen or C₁₋₄alkyl, or a pharmaceutically acceptable salt thereof;
(XII) compounds which have the structure 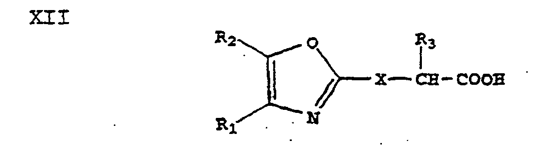 Where X is O or S;
R₁ is H, phenyl or phenyl substituted with F, Cl or Br or alkoxy,
R₂ is H, alkyl, phenyl or phenyl substituted with F, Cl or Br or alkoxy, and
R₃ is H or alkyl;
(XIII) 2-benzyloxypyrimidine derivatives having the following structure 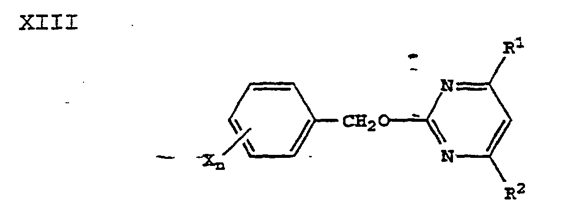 wherein
R¹ and R² are each independently H, a halogen, hydroxyl, C₁-C₄ alkyl, C₁-C₄ haloalkyl, C₃-C₅ alkenyl, C₃-C₅ alkynyl, C₁-C₄ alkoxy, C₁-C₄ haloalkoxy, C₃-C₅ alkenyloxy, C₃-C₅ alkynyloxy, C₁-C₄ alkylthio, or phenyl, with the proviso that at least one of R¹ and R² must be hydroxyl;
n is an integer of 0 to 5; and
each X which may be identical or different if n is greater than 1, is a halogen, C₁-C₄ alkyl, C₁-C₄ haloalkyl, C₁-C₄ alkoxy, C₁-C₄ alkylthio, C₇-C₉ aralkyloxy, phenyl, hydroxymethyl, hydroxycarbonyl, C₁-C₄ alkoxycarbonyl, or nitro;
(XIV) dihydro(alkylthio)-(naphthylmethyl)-oxopyrimidines which have the structures 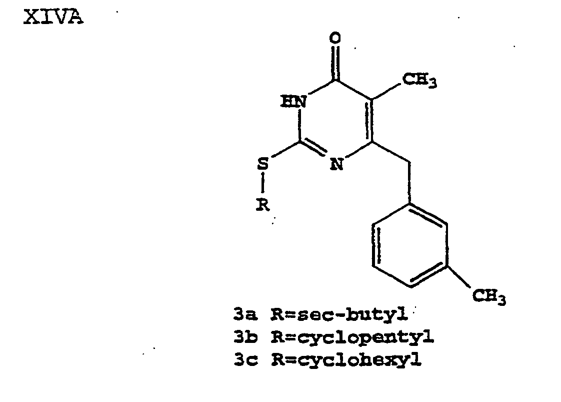 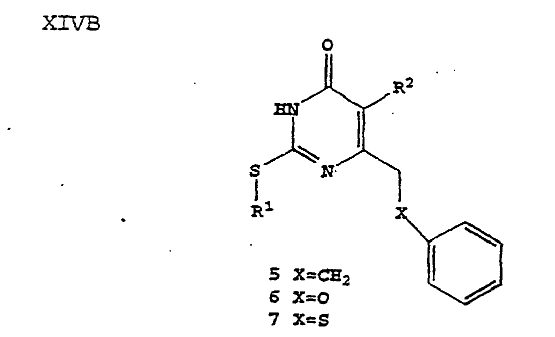 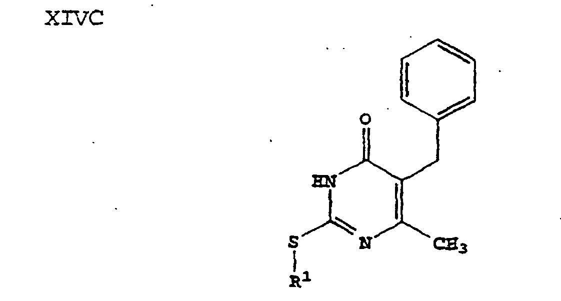 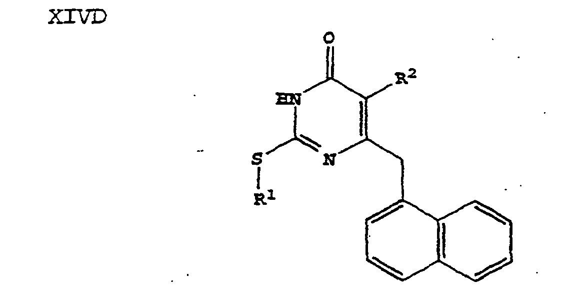 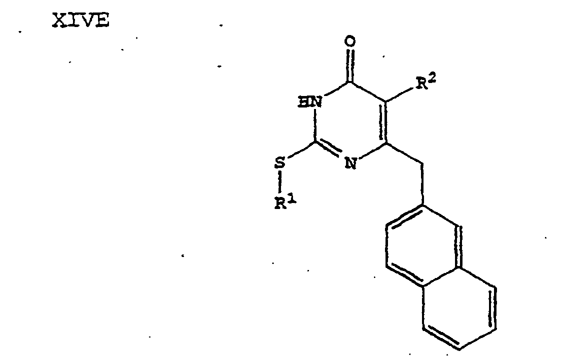 R¹ = sec-butyl, cyclopentyl, cyclohexyl;
R² = H, CH₃, including tautomers of the above;
(XVI) α-substituted pyrimidine-thioalkyl and alkylether compounds which have the structure 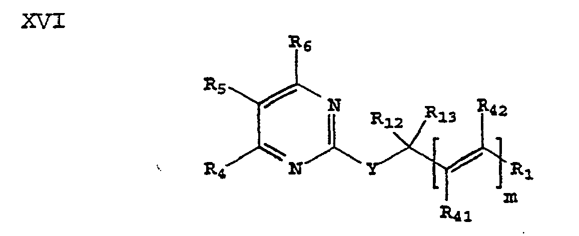 where m is 0 or 1;
R¹ is selected from -CO₂R₅₃, -CONR₅₄R₅₅, 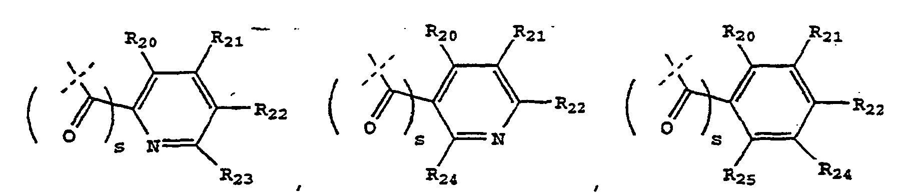 where s is 0 or 1, and R₂₀, R₂₁, R₂₂, R₂₃, R₂₄, and R₂₅ are the same or different and are selected from -H, C₁-C₆ alkyl, C₁-C₆ alkenyl, C₁-C₆ alkoxy, C₁-C₆ alkylthio, C₃-C₈ cycloalkyl, -CF₃, -NO₂, -halo, -OH, -CN, phenyl, phenylthio, -styryl, -CO₂ (R₃₁), -CON (R₃₁) (R₃₂), -CO (R₃₁), - (CH₂)ₙ-N(R₃₁)(R₃₂), -C (OH) (R₃₁(R₃₃), - (CH₂)ₙN(R₃₁) (CO (R₃₃)), (CH₂) ₙN(R₃₁) (SO₂(R₃₃)), or where R₂₀ and R₂₁, or R₂₁ and R₂₂, or R₂₂ and R₂₃ are taken together to form a five or six-membered saturated or unsaturated ring containing 0 or 1 oxygen, nitrogen or sulfur, where the unsaturated ring may be optionally substituted with 1, 2 or 3, C₁-C₆ alkyl, C₁-C₆ alkoxy, -OH, -CH₂OH, -(CH₂)ₙ-N(R₃₁) (R₃₂), -C₃-C₈ cycloalkyl, -CF₃, -halo, CO₂ (R₃₁), -CON(R₃₁) (R₃₂), -CO(R₃₁), -(CH₂)ₙN(R₃₁) (CO(R₃₃)), -(CH₂)ₙN(R₃₁) (SO₂(R₃₃)). -CN, -CH₂CF₃ or -CH(CF₃)₂, or phenyl and the saturated ring may be optionally substituted with 1, 2 or 3, -C₁-C₆ alkyl, -C₁-C₆ alkoxy, -OH, -CH₂OH or -(CH₂)ₙ-N(R₃₁)(R₃₂) or one oxo (=O);
where n is 0-3 and R₃₁, R₃₂ and R₃₃ are the same or different and are selected from
-H,
C₁-C₆ alkyl,
phenyl optionally substituted with 1, 2 or 3 -halo, C₁-C₆ alkyl, C₁-C₆ alkoxy, -CF₃, -OH or -CN,
or where R₃₁ and R₃₂ taken together with the attached nitrogen to form a ring selected from -pyrrolidnyl, piperidinyl, -4-morpholinyl, -4-thiomorpholinyl, -4-piperazinyl, -4-(1-C₁-C₆alkyl)piperazinyl, or a member selected from
1-cyclohexenyl, 2-pyrimidinyl, 4-pyrimidinyl, 5-pyrimidinyl, 2-imidazolyl, 4-imidazolyl, 2-benzothiazolyl, 2-benzoxazolyl, 2-benzimidazolyl, 2-oxazolyl, 4-oxazolyl, 2-thiazolyl, 3-isoxazolyl, 5-isoxazolyl, 5-methyl-3-isoxazolyl, 5-phenyl-3-isoxazolyl, 4-thiazolyl, 3-methyl-2-pyrazinyl, 5-methyl-2-pyrazinyl, 6-methyl-2-pyrazinyl, 5-chloro-2-thienyl, 3-furyl, benzofuran-2-yl, benzothien-2-yl, 2H-1-benzopyran-3-yl, 2,3-dihydrobenzopyran-5-yl, 1-methylimidazol-2-yl, quinoxalin-2-yl, piperon-5-yl, 4,7-dichlorobenzoxazol-2-yl, 4,6-dimethylpyrimidin-2-yl, 4-methylpyrimidin-2-yl, 2,4-dimethylpyrimidin-6-yl, 2-methylpyrimidin-4-yl, 4-methylpyrimidin-6-yl, 6-chloropiperon-5-yl, 5-chloroimidazol[1,2-a]pyridin-2-yl, 1-H-inden-3-yl, 1-H-2-methyl-inden-2-yl, 3,4-dihydronaphth-1-yl, S-4-isopropenylcyclohexen-1-yl or 4-dihydronaphth-2-yl;
where R₅₃ is selected from -H, C₁-C₆alkyl, C₃-C₅cycloalkyl, phenyl (optionally substituted with 1, 2, or 3 -halo, C₁-C₆ alkyl , C₁-C₆ alkoxy, -CF₃ , -OH, -CN), or a five or six-membered unsaturated ring containing 0 or 1 oxygen, nitrogen or sulfur, where the unsaturated ring may be optionally substituted with -H, C₁-C₆ alkyl, C₁-C₆ alkoxy, -OH, -CH₂OH, or -(CH₂)ₙ-N(R₃₁)(R₃₂);
where R₅₄ and R₅₅ being the same or different are selected from -H, C₁-C₆ alkyl, allyl, or phenyl (optionally substituted with 1, 2 or 3 -halo, C₁-C₆ alkyl, C₁-C₆ alkoxy or -CF₃), or taken together with the attached nitrogen to form a ring selected from -pyrrolidinyl, -piperidinyl, -4-morpholinyl, -4-thiomorpholinyl, -4-piperazinyl, -4-(1-C₁-C₆alkyl)piperazinyl;
R₄₁ and R₄₂, being the same or different, are selected from -H and C₁-C₄ alkyl;
R₁₂ is selected from -H, C₁-C₆ alkyl, -C₃-C₆ cycloalkyl, -CN, -C(O)NH₂, -C(O)N(C₁-C₆alkyl)(C₁-C₆alkyl), - CO₂H, -CO₂(C₁-C₆alkyl), -CH₂OH, -CH₂NH₂ or -CF₃;
R₁₃ is selected from -H, C₁-C₆ alkyl or -CF₃; Y is selected from -S-, -S(O)-, -S(O)₂, or -O-;
R₄ is -OH;
R₅ is selected -H, -C₂H₄OH- -C₂H₄-O-TBDMS, halo, -C₃-C₆ cycloalkyl, C₁-C₃ alkoxy, -CH₂CH₂Cl or C₁-C₄ alkyl, with the proviso that R₅ is not isobutyl;
or, when R₆ is hydroxyl, R₄ and R₅ are taken together to form a five or six-memebered saturated or unsaturated ring which together with the pyrimidine ring form the group consisting of 7H-pyrrolo[2,3-d]pyrimidine, 5,6-dihydro-7H-pyrrolo[2,3-d]pyrimidine, furo[2,3-d]pyrimidine, 5,6-dihydro-furo[2,3-d]pyrimidine, thieno[2,3-d]pyrimidine, 5,6-dihydro-thieno[2,3-d]pyrimidine, 1H-pyrazolo[3,4-d]pyrimidine, 1H-purine, pyrimido[4,5-d]pyrimidine, pteridine, pyrido[2,3-d]pyrimidine, or quinazoline, where the unsaturated ring may be optionally substituted with 1, 2 or 3, C₁-C₆ alkyl C₁-C₆ alkoxy, -OH, -CH₂OH, or -(CH₂)ₙ-N(R₃₁) (R₃₂), -C₃-C₈ cycloalkyl, -CF₃, -halo, -CO₂(R₃₁), - CON(R₃₁) (R₃₂), -CO(R₃₁), - (CH₂)ₙN(R₃₁) (CO (R₃₃)), - (CH₂)ₙN(R₃₁) (SO₂(R₃₃)), and the saturated ring may be optionally substituted with 1, 2 or 3, -C₁-C₆ alkyl, C₁-C₆ alkoxy, -OH, -CH₂OH, or -(CH₂)ₙ-N(R₃₁)(R₃₂) or one oxo (=O); and
R₆ is selected from -H, -OH, halo, -CN, -CF₃, - CO₂(R₆₁), -C(O)R₆₁ or -C(O)N(R₆₁) (R₆₂) where R₆₁ and R₆₂ are the same or different and are selected from
-H,
C₁-C₆ alkyl,
phenyl optionally substituted with 1, 2 or 3 -halo, C₁-C₆ alkyl, C₁-C₆ alkoxy, -CF₃, -OH, -CN,
or where R₆₁ and R₆₂ taken together with the attached nitrogen to form a ring selected from -pyrrolidinyl, - piperidinyl, -4-morpholinyl, -4-thiomorpholinyl, -4-piperazinyl, or -4-(C₁-C₆ alkyl)piperazinyl;
pharmaceutically acceptable salts, hydrates, N-oxides and solvates thereof;
(XVII) compounds which have the structure 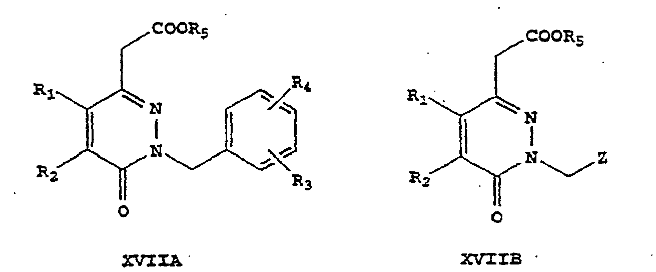
where R₁ and R₂ are H, alkyl, aryl or arylalkyl, where the alkyl can include as substituents halogen, CF₃, CH₃O, CH₃S, NO₂, or R₁ and R₂ with the carbons to which they are attached, can form methylenedioxy, or
R₁ and R₂ can form a C₃-C₇ non-aromatic ring; or a heterocycle which can be pyridine, pyrazine, pyrimidine, pyridazine, indol, or pyrazole, or an oxygen containing heterocycle which can be pyran or furan, or a sulfur containing heterocycle which can be thiopyran, or thiophene; the heterocycles being optionally substituted with halogen or alkyl,
R₃ and R₄ are H, alkyl, halogen, CF₃, CH₃O, CH₃S or NO₂ or R₃ and R₄ with the carbons to which they are attached can form a methylenedioxy group,
R₅ is H, and
Z is a heterocycle which can be pyridine, thiazole, benzothiazole, benzimidazole or quinoline, which Z group can optionally be substituted with halogen or alkyl.

15. The use as defined in Claim 1 wherein the aP2 inhibitor has the structure 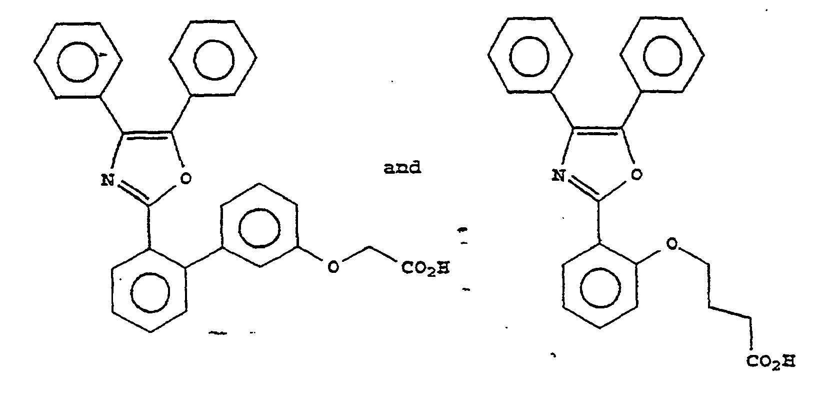 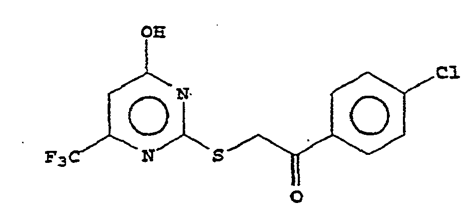 or 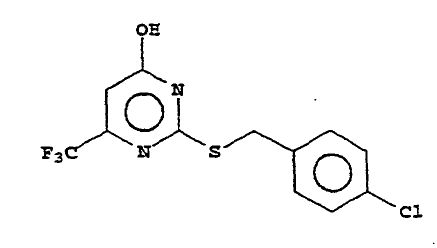 or

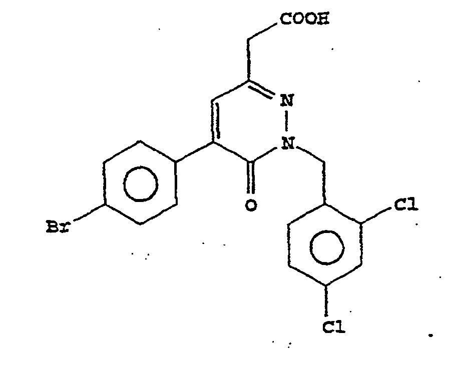

16. A pharmaceutical combination comprising an aP2 inhibitor and another type antidiabetic agent.

17. The combination as defined in Claim 16 wherein the antidiabetic agent is a biguanide, a sulfonyl urea, a glucosidase inhibitor, a thiazolidinedione, an insulin sensitizer, a glucagon-like peptide-1 (GLP-1), insulin, a PPAR α/γ dual agonist and/or a meglitinide.

18. The combination as defined in Claim 16 wherein the antidiabetic agent is metformin, glyburide, glimepiride, glipyride, glipizide, chlorpropamide, gliclazide, acarbose, miglitol, troglitazone, insulin, KRP-297, repaglinide and/or nataglinide.

19. The combination as defined in Claim 16 wherein the aP2 inhibitor is present in a weight ratio to the antidiabetic agent within the range from about 0.01 to about 100:1.

20. A pharmaceutical composition as defined in claim 16 for treating insulin resistance, diabetes, obesity, hyperglycemia, hyperinsulinemia, or elevated blood levels of free fattv acids, or glycerol, or hypertriglyceridemia.

## Patentansprüche

1. Verwendung eines aP2-Hemmers für die Herstellung einer pharmazeutischen Zusammensetzung zum Behandeln von Diabetes, Insulinresistenz, Obesitas, Hyperglykämie, Hyperinsulinämie oder erhöhten Fettsäuren oder Glycerin oder Hypertriglyceridämie.

2. Verwendung nach Anspruch 1, wobei der aP2-Hemmer an dem aP2-Protein bindet und dessen Funktion hemmt und/oder dessen Fähigkeit zum Binden freier Fettsäuren.

3. Verwendung nach Anspruch 1, wobei der aP2-Hemmer eine Wasserstoff-gebundene Donator- oder Akzeptorgruppe enthält und direkt oder über einem intervenierenden Wassermolekül entweder durch ionische oder Wasserstoffbindungs-Wechselwirkungen mit einem, zwei oder drei der drei Aminosäurereste, bezeichnet als Arg 106, Arg 126 und Tyr 128, in Human-aP2 im Inneren des aP2-Proteins in Wechselwirkung steht.

4. Verwendung nach Anspruch 3, wobei die Wasserstoff-gebundene Donatorgruppe oder Akzeptorgruppe eine saure Beschaffenheit hat.

5. Verwendung nach Anspruch 3, wobei der aP2-Hemmer einen zusätzlichen Substituenten enthält, der an (in) einer diskreten Tasche im Inneren des aP2-Proteins bindet und/oder mit dieser in Wechselwirkung steht, wobei das aP2-Protein im Human-aP2 grob festgelegt ist durch die Aminosäurereste Phe 16, Tyr 19, Met 20, Val 23, Val 25, Ala 33, Phe 57, Thr 74, Ala 75, Asp 76, Arg 78.

6. Verwendung nach Anspruch 5, wobei der zusätzliche Substituent in dem aP2-Hemmer eine hydrophobe Beschaffenheit hat.

7. Verwendung nach Anspruch 5, worin der Abstand durch den Raum von der Wasserstoffgebundenen Donator/Akzeptorgruppe und der zusätzlichen Substituentengruppe in dem aP2-Hemmer innerhalb des Abstandes von etwa 7 bis etwa 15 Angström liegt.

8. Verwendung nach Anspruch 1, wobei Diabetes Typ II behandelt wird.

9. Verwendung nach Anspruch 1, wobei der aP2-Hemmer in Form eines pharmazeutisch duldbaren Salzes davon oder in Form eines Prodrug-Esters eingesetzt wird.

10. Verwendung nach Anspruch 1, wobei in den aP2-Hemmer ein Oxazol- oder ein analoger Ring einbezogen ist, ein Pyrimidin-Derivat oder ein Pyridazinon-Derivat.

11. Verwendung nach Anspruch 10, wobei der aP2-Hemmer ein substituiertes Benzoyl- oder Biphenyl-2-oxazol-alkansäure-Derivat ist, ein Oxazol-Derivat, ein 2-Thio-4,5-diphenyloxazol-S-Derivat, ein Phenyl-heterocyclisches Oxazol-Derivat, ein Diaryloxazol-Derivat, ein 4,5-Diphenyloxazol-Derivat, ein Oxazolcarbonsäure-Derivat, ein Phenyloxazolyloxazol-Derivat oder ein 2-(4,5-Diaryl)-2-oxazolyl-substituiertes Phenoxyalkansäure-Derivat.

12. Verwendung nach Anspruch 10, wobei der aP2-Hemmer ein 2-Benzoyloxypyrimidin-Derivat ist, ein Dihydro(alkylthio)(naphthylmethyl)oxypyrimidin-Derivat, ein Thiouracil-Derivat oder ein αsubstituiertes Pyrimidinthiolalkyl- oder Alkylether-Derivat.

13. Verwendung nach Anspruch 10, wobei der aP2-Hemmer ein Pyridazinonessigsäure-Derivat ist.

14. Verwendung nach Anspruch 10, wobei der aP2-Hemmer ist:
(I) ein substituiertes Benzoylbenzol oder ein Biphenylalkansäure-Derivat mit der Struktur:
I A (CH₂)ₙO-B
worin
A eine Gruppe mit der Formel ist: 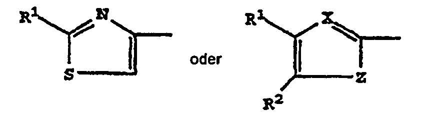 worin
X -N- ist oder 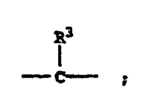 Z ist: 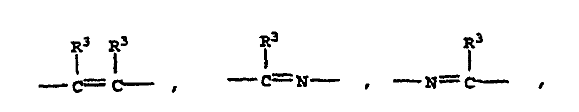 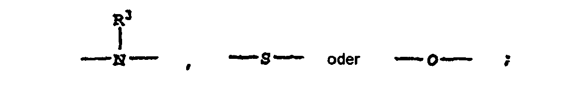 R¹ ist Wasserstoff, niederes Alkyl oder Phenyl;
R² ist Wasserstoff oder niederes Alkyl; oder
R¹ und R² bilden zusammengenommen einen Benzolring mit der Maßgabe, dass, wenn X -N- ist, Z nicht 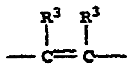 sein kann;
R³ ist Wasserstoff oder niederes Alkyl;
n beträgt 1 bis 2;
B ist: 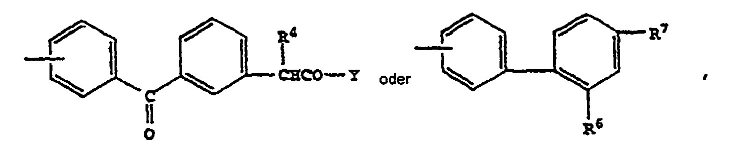 worin:
Y OR⁵ ist oder N(OH)R⁸;
R⁴ und R⁵ sind jeweils unabhängig Wasserstoff oder niederes Alkyl;
R⁶ ist Wasserstoff, Halogen oder Nitro;
R⁷ ist: 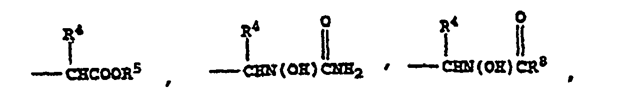 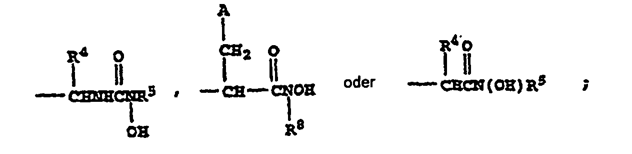
R⁸ ist niederes Alkyl;
m beträgt 0 bis 3;
oder pharmakologisch duldbare Salze davon;
(II) Oxazol-Derivate, welche die Struktur haben: 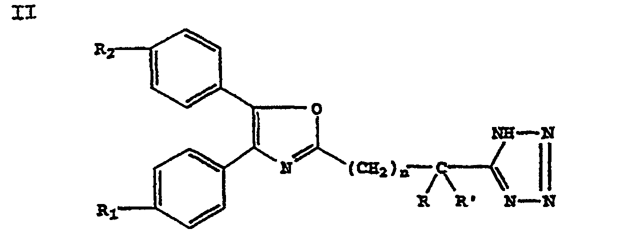 worin:
R und R' identisch oder verschieden sind und ein Wasserstoffatom darstellen oder einen AlkylRest, der 1 bis 2 Kohlenstoffatome enthält,
R₁ und R₂ sind identisch oder verschieden und stellen Wasserstoff oder Halogenatome oder Alkyloxy-Reste dar, worin der Alkyl-Abschnitt 1 bis 4 Kohlenstoffatome in einer geraden oder verzweigten Kette enthält, und
n ist gleich 3 bis 6; sowie deren Salze;
(III) 2-Thiol-4,5-diphenyloxazol-S-Derivate, welche die Struktur haben: 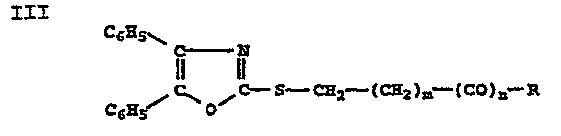 worin m 0, 1 oder 2 beträgt, n ist 1 und R stellt Hydroxy dar, Alkoxy oder Amino; sowie pharmazeutisch duldbare Anlagerungssalze davon;
(IV) Azol-Derivate der Struktur 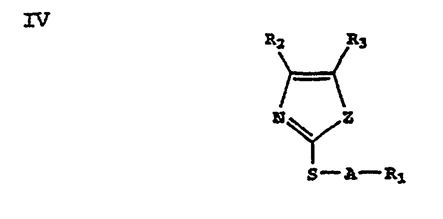 worin R₁ Carboxyl ist, verestertes Carboxyl oder eine andere funktionell modifizierte CarboxylGruppe; R₂ und R₃ sind jeweils Aryl mit bis zu 10 Kohlenstoffatomen; A ist CₙH₂ₙ, worin n eine ganze Zahl zwischen 1 und 10 einschließlich ist; und Z ist O oder S; sowie physiologisch duldbare Salze davon;
(V) Phenyl-heterocyclische Oxazol-Derivate, welche die Struktur haben: 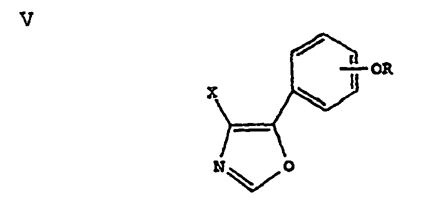 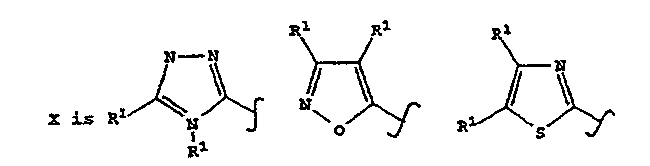 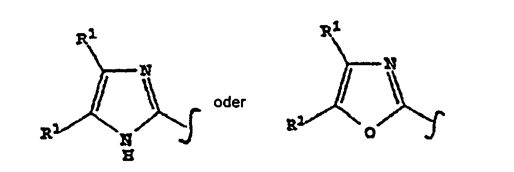 R ist CH₂R²;
R¹ ist Ph oder Th;
R² ist 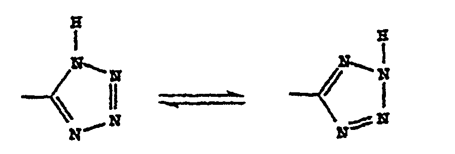 CO₂R³; und
R³ ist H oder C₁-C₄-niederes Alkyl
oder pharmazeutisch duldbare Salze davon;
(VI) Diaryloxazol-Derivate, welche die Struktur haben: 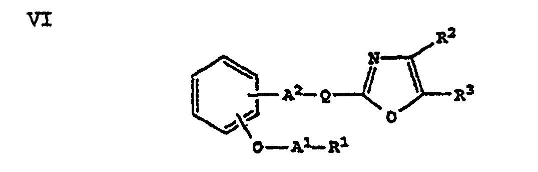 worin R¹ Carboxy oder geschütztes Carboxy ist,
R² ist Aryl,
R³ ist Aryl,
A¹ ist niederes Alkylen,
A² ist eine Bindung oder niederes Alkylen und
-Q- ist 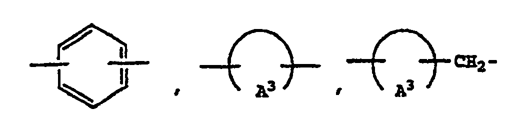 oder 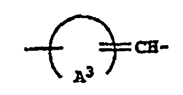 (worin 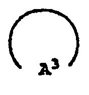 ein Cyclo(niederes)alkan oder Cyclo(niederes)alken ist,
wobei jedes von diesen einen/mehrere geeignete Substituenten hat;
(VII) 4,5-Diphenyloxazol-Derivate, welche die Struktur haben: 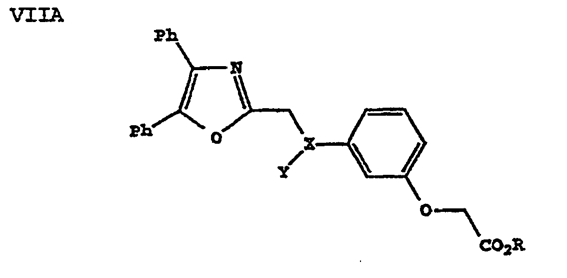 worin:
R H ist oder C₁ bis C₅-niederes Alkyl
X ist N oder CH,
Y ist H oder CO₂R¹ oder COR² mit der Maßgabe, dass, wenn X CH ist, Y nicht H ist,
R¹ ist C₁ bis C₅-niederes Alkyl oder Phenylmethyl und
R² ist C₁ bis C₅-Alkyl; 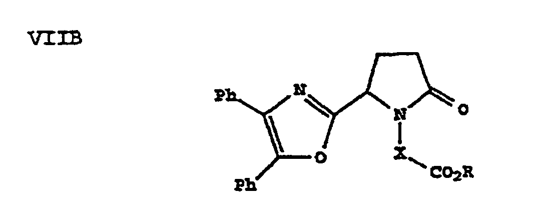
worin:
R H ist oder C₁ bis C₅-niederes Alkyl,
X ist (CH₂)ₙ oder in para- oder meta-Stellung substituiertes Phenyl, worin der Substituent OR² ist, R² ist C₁ bis C₅ Alkyl und
n ist eine ganze Zahl von 4 bis 8,
sowie pharmazeutisch duldbare Salze davon;
(VIII) Oxazolcarbonsäure-Derivate, welche die Struktur haben: 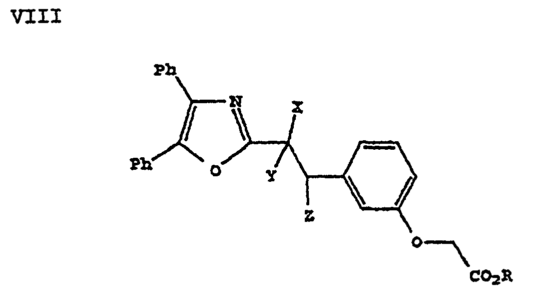 worin:
Y und Z unabhängig Wasserstoff sind oder gemeinsam eine Bindung bilden;
X ist CN, CO₂R¹ oder CONR²R³;
R und R¹ sind unabhängig oder gemeinsam H, Na oder C₁ bis C₅-niederes Alkyl;
R² und R³ sind unabhängig oder gemeinsam H oder C₁ bis C₅-niederes Alkyl;
oder Alkalimetallsalz davon;
(IX) Phenyloxazolyloxazol-Derivate, welche die Struktur haben: 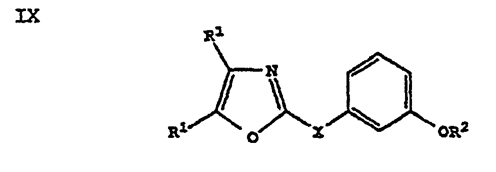 worin
X 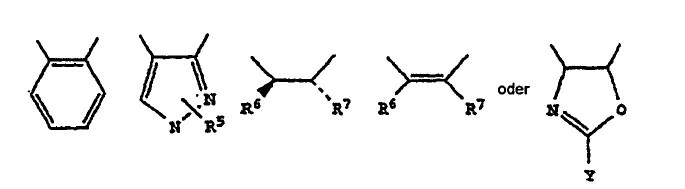 ist,
Y ist CH₃, Ph oder OH mit der Maßgabe, dass, wenn Y OH ist, die Verbindung in einer tautaumeren Keto-Enol-Form existiert: 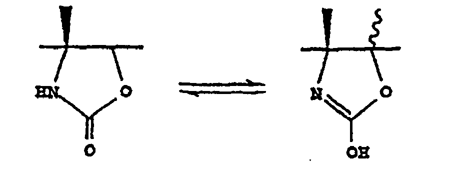 R¹ ist Ph oder Th;
R² ist CH₂R³;
R³ ist CO₂R⁴;
R⁴ ist H oder C₁ bis C₅-niederes Alkyl;
R⁵ ist H oder CH₃; R⁶ ist OHCHN oder H₂N und
R⁷ ist H oder OH;
oder ein pharmazeutisch duldbares Salz davon;
(X) 2-(4,5-Diaryl)-2-Oxazolyl-substituierte Phenoxyalkansäuren und Ester, welche die Struktur haben: 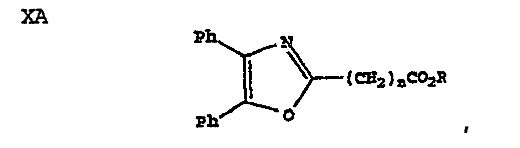 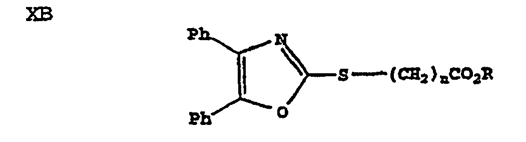 (worin n 7 bis 9 beträgt und R Wasserstoff ist oder niederes Alkyl; oder wenn R Wasserstoff ist, das Alkalimetallsalz davon), 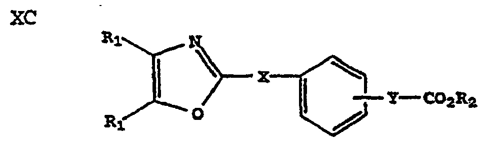 oder 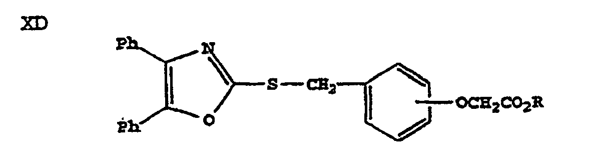 worin:
R₁ Phenyl oder Thienyl ist;
R₂ ist Wasserstoff, niederes Alkyl oder gemeinsam mit CO₂ Tetrazol-1-yl;
X ist eine zweiwertige verknüpfende Gruppe, ausgewählt aus der Gruppe, bestehend aus: CH₂CH₂, CH=CH und CH₂O;
Y ist eine zweiwertige verknüpfende Gruppe, die in 3- oder 4-Phenyl-Stellung angebracht ist, ausgewählt aus der Gruppe, bestehend aus: OCH₂, CH₂CH₂ und CH=CH;
oder wenn R₂ Wasserstoff ist, ein Alkalimetallsalz davon;
(XI) substituierte 4,5-Diaryl-Heterocyclen, welche die Formel haben: 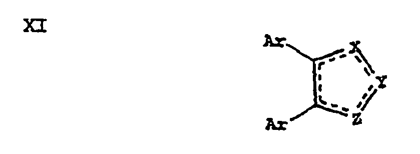 worin:
jede Gruppe Ar gleich oder verschieden ist und wahlweise substituiertes Phenyl oder wahlweise substituiertes Heteroaryl ist;
X ist Stickstoff oder CR¹;
Y ist Stickstoff, N(CH₂)ₙA oder C(CH₂)ₙA;
Z ist Stickstoff, Sauerstoff oder N(CH₂)ₙA, wobei die gepunktete Linie das wahlweise Vorhandensein einer Doppelbindung angibt, um auf diese Weise einen vollständig ungesättigten heterocyclischen Ring zu bilden;
R¹ ist Wasserstoff, C₁₋₄-Alkyl, wahlweise substituiertes Phenyl oder wahlweise substituiertes Heteroaryl;
n beträgt 4 bis 12 und
A ist CO₂H oder eine Gruppe, die zu CO₂H hydrolysierbar ist, 5-tetrazolyl, SO₃H, P(O)(OR)₂, P(O)(OH)₂ oder P(O)(R)(OR), worin R Wasserstoff ist oder C₁₋₄-Alkyl oder ein pharmazeutisch duldbares Salz davon;
(XII) Verbindung, welche die Struktur hat: 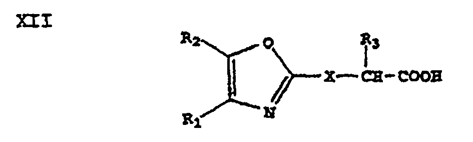 worin X O oder S ist;
R₁ ist H, Phenyl oder Phenyl, das substituiert ist mit F, Cl oder Br, oder Alkoxy,
R₂ ist H, Alkyl, Phenyl oder Phenyl, das substituiert ist mit F, Cl oder Br, oder Alkoxy und
R₃ ist H oder Alkyl;
(XIII) 2-Benzyloxypyrimidin-Derivate, welche die folgende Struktur haben: 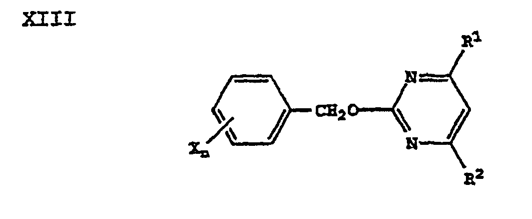 worin:
R¹ und R² jeweils unabhängig H sind, ein Halogen, Hydroxyl, C₁-C₄-Alkyl, C₁-C₄-Halogenalkyl, C₃-C₅-Alkenyl, C₃-C₅-Alkinyl, C₁-C₄-Alkoxy, C₁-C₄-Halogenalkoxy, C₃-C₅-Alkenyloxy, C₃-C₅-Alkinyloxy, C₁-C₄-Alkylthio oder Phenyl mit der Maßgabe, dass mindestens eines von R¹ und R² Hydroxyl sein muss;
n ist eine ganze Zahl von 0 bis 5; und
jedes X, das identisch oder verschieden sein kann, wenn n größer ist als 1, ist ein Halogen, C₁-C₄-Alkyl, C₁-C₄-Halogenalkyl, C₁-C₄-Alkoxy, C₁-C₄-Alkylthio, C₇-C₉-Aralkyloxy, Phenyl, Hydroxymethyl, Hydroxycarbonyl, C₁-C₄-Alkoxycarbony) oder Nitro;
(XIV) Dihydro(alkylthio)-(naphthylmethyl)-oxopyrimidine, welche die Strukturen haben: 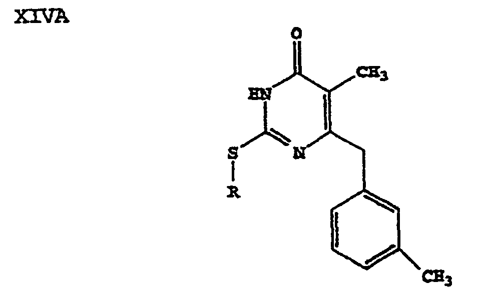 3a R=*sec*-Butyl
3b R=Cyclopentyl
3c R=Cyclohexyl 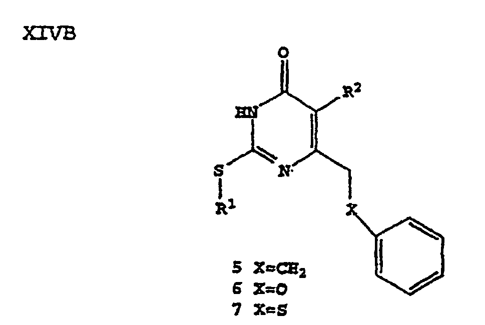 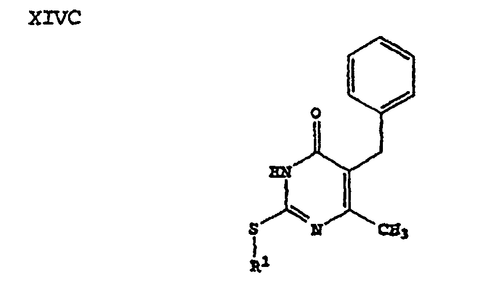 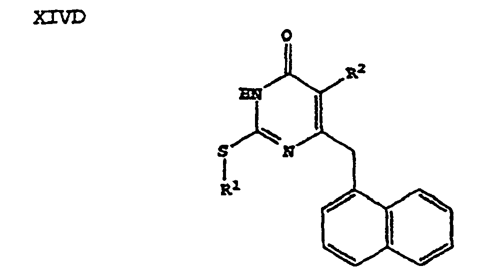 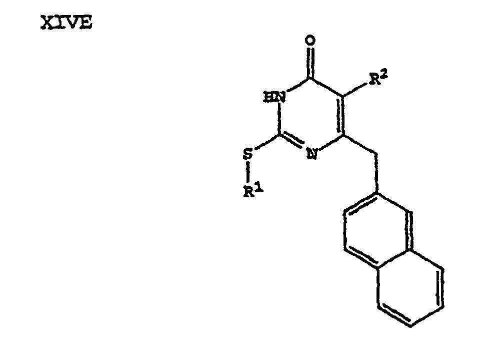 R¹ = *sec*-Butyl, Cyclopentyl, Cyclohexyl;
R²= H, CH₃, einschließend Tautomere der Vorgenannten;
(XVI) α-substituiertes Pyrimidinthioalkyl und Alkylether-Verbindungen, welche die Struktur haben: 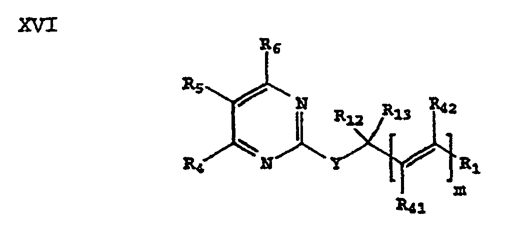 worin m 0 oder 1 beträgt;
R¹ ist ausgewählt aus -CO₂R₅₃, -CONR₅₄R₅₅, 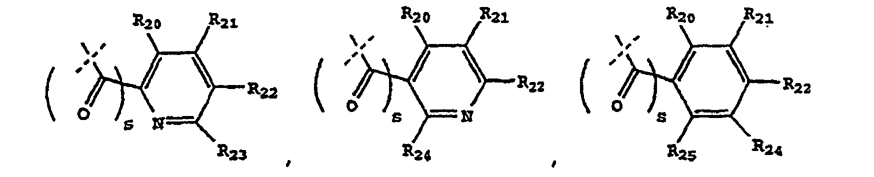 worin s 0 oder 1 ist und R₂₀, R₂₁, R₂₂, R₂₃, R₂₄ und R₂₅ sind gleich oder verschieden und sind ausgewählt aus: -H, C₁ bis C₆-Alkyl, C₁ bis C₆-Alkenyl, C₁ bis C₆-Alkoxy, C₁ bis C₆-Alkylthio, C₃ bis C₈-Cycloalkyl, -CF₃, -NO₂, -Halogen, -OH, -CN, Phenyl, Phenylthio, -Styryl, -CO₂(R₃₁), -CON(R₃₁)(R₃₂), - CO(R₃₁), -(CH₂)ₙ-N(R₃₁)(R₃₂), -C(OH)(R₃₁)(R₃₃), -(CH₂)ₙ-N(R₃₁) (CO(R₃₃)), -(CH₂)ₙ-N(R₃₁) (SO₂(R₃₃)) oder worin R₂₀ und R₂₁ oder R₂₁ und R₂₂ oder R₂₂ und R₂₃ zusammengenommen einen fünfgliedrigen oder sechsgliedrigen, gesättigten oder ungesättigten Ring bilden, der 0 oder 1 Sauerstoffatome, Stickstoffatome oder Schwefelatome enthält, wobei der ungesättigte Ring wahlweise substituiert sein kann mit 1, 2 oder 3 - C₁ bis C₆-Alkyl, C₁ bis C₆-Alkoxy, -OH, -CH₂OH, -(CH₂)ₙ-N(R₃₁)(R₃₂), C₃ bis C₈-Cycloalkyl, -CF₃, - Halogen, -CO₂(R₃₁), -CON(R₃₁)(R₃₂), -CO(R₃₁), -(CH₂)ₙ-N(R₃₁) (CO(R₃₃)), -(CH₂)ₙ-N(R₃₁) (SO₂(R₃₃)),-CN, -CH₂CF₃ oder -CH(CF₃)₂ oder Phenyl, und wobei der gesättigte Ring wahlweise substituiert sein kann mit 1, 2 oder 3 -C₁-C₆-Alkyl, -C₁-C₆-Alkoxy, -OH, -CH₂OH oder -(CH₂)ₙ-N(R₃₁)(R₃₂) oder eines Oxo (=0);
worin n 0 bis 3 beträgt und R₃₁, R₃₂ und R₃₃ gleich oder verschieden sind und ausgewählt sind aus:
-H,
C₁-C₆-Alkyl,
Phenyl, wahlweise substituiert mit 1, 2 oder 3 Halogen, C₁-C₆-Alkyl, C₁-C₆-Alkoxy, -CF₃, -OH oder -CN,
oder worin R₃₁ und R₃₂ zusammengenommen mit dem anhängenden Stickstoff einen Ring bilden, ausgewählt aus: -Pyrrolidinyl, -Pyperidinyl, -4-Morpholinyl, -4-Thiomorpholinyl, -4-Piperazinyl, -4-(1-C₁-C₆-Alkyl)piperazinyl oder ein Vertreter, der ausgewählt ist aus:
1-Cyclohexenyl, 2-Pyrimidinyl, 4-Pyrimidinyl, 5-Pyrimidinyl, 2-Imidazolyl, 4-Imidazolyl, 2-Benzothiazolyl, 2-Benzoxazolyl, 2-Benzimidazolyl, 2-Oxazolyl, 4-Oxazolyl, 2-Thiazolyl, 3-Isoxazolyl, 5-Isoxazolyl, 5-Methyl-3-isoxazolyl, 5-Phenyl-3-isoxazolyl, 4-Thiazolyl, 3-Methyl-2-pyrazinyl, 5-Methyl-2-pyrazinyl, 6-Methyl-2-pyrazinyl, 5-Chlor-2-thienyl, 3-Furyl, Benzofuran-2-yl, Benzothien-2-yl, 2H-1-benzopyran-3-yl, 2,3-Dihydrobenzopyran-5-yl, 1-Methylimidazol-2-yl, Chinoxalin-2-yl, Piperon-5-yl, 4,7-Dichlorbenzoxazol-2-yl, 4,6-Dimethylpyrimidin-2-yl, 4-Methylpyrimidin-2-yl, 2,4-Dimethylpyrimidin-6-yl, 2-Methylpyrimidin-4-yl, 4-Methylpyrimidin-6-yl, 6-Chlorpiperon-5-yl, 5-Chlorimidazol[1,2-a]pyridin-2-yl, 1-H-Inden-3-yl, 1-H-2-methyl-inden-2-yl, 3,4-Dihydronaphth-1-yl, S-4-Isopropenylcyclohexen-1-yl oder 4-Dihydronaphth-2-yl;
worin R₅₃ ausgewählt aus -H, C₁ bis C₆-Alkyl, C₃ bis C₅-Cycloalkyl, Phenyl (wahlweise substituiert mit 1, 2 oder 3-Halogen, C₁ bis C₆-Alkyl, C₁ bis C₆-Alkoxy, -CF₃, -OH, -CN) oder ein fünf-oder sechsgliedriger ungesättigter Ring, der 0 oder 1 Sauerstoff, Stickstoff oder Schwefel enthält, wobei der ungesättigte Ring wahlweise substituiert sein kann mit -H, C₁ bis C₆-Alkyl, C₁ bis C₆-Alkoxy, -OH, - CH₂OH oder -(CH₂)ₙ-N(R₃₁)(R₃₂);
worin R₅₄ und R₅₅ gleich oder verschieden sind und ausgewählt sind aus: -H, C₁ bis C₆-Alkyl, Allyl oder Phenyl (wahlweise substituiert mit 1, 2 oder 3 -Halogen, C₁-C₆-Alkyl, C₁-C₆-Alkoxy oder -CF₃) oder zusammengenommen mit dem anhängenden Stickstoff ein Ring bilden können, ausgewählt aus: - Pyrrolidinyl, -Piperidinyl, -4-Morpholinyl, -4-Thiomorpholinyl, -4-Piperazinyl, -4-(1-C₁-C₆-Alkyl)piperazinyl;
R₄₁ und R₄₂ sind gleich oder verschieden und ausgewählt aus -H und C₁-C₄-Alkyl;
R₁₂ ist ausgewählt aus -H, C₁ bis C₆-Alkyl, -C₃ bis C₆-Cycloalkyl, -CN, -C(O)NH₂, -C(O)N(C₁-C₆-Alkyl)(C₁-C₆-Alkyl), -CO₂H, -CO₂(C₁-C₆-Alkyl), -CH₂OH, -CH₂NH₂ oder -CF₃;
R₁₃ ist ausgewählt aus -H, C₁ bis C₆-Alkyl oder -CF₃;
Y ist ausgewählt aus -S-, -S(O)-, -S(O)₂ oder -O-;
R₄ ist -OH;
R₅ ist ausgewählt aus: -H, -C₂H₄OH₅, -C₂H₄-O-TBDMS, Halogen, -C₃-C₆-Cycloalkyl, C₁-C₆-Alkoxy, -CH₂CH₂Cl oder C₁-C₆-Alkyl mit der Maßgabe, dass R₅ nicht Isobutyl ist;
oder, wenn R₆ Hydroxyl ist, bilden R₄ und R₅ zusammengenommen einen fünfgliedrigen oder sechsgliedrigen, gesättigten oder ungesättigten Ring, der zusammen mit dem Pyrimidin-Ring die Gruppe bildet, bestehend aus: 7H-Pyrrolo[2,3-d]pyrimidin, 5,6-Dihydro-7H-pyrrolo[2,3-d]pyrimidin, Furo[2,3-d]pyrimidin, 5,6-Dihydro-furo[2,3-d]pyrimidin, Thieno[2,3-d]pyrimidin, 5,6-Dihydro-thieno[2,3-d]pyrimidin, 1H-Pyrazolo[3,4-d]pyrimidin, 1H-Purin, Pyrimido[4,5-d]pyrimidin, Pteridin, Pyrido[2,3-d]pyrimidin oder Chinazolin, wobei der ungesättigte Ring wahlweise substituiert sein kann mit 1, 2 oder 3 - C₁ bis C₆-Alkyl, C₁ bis C₆-Alkoxy, -OH, -CH₂OH oder -(CH₂)ₙ-N(R₃₁)(R₃₂), -C₃-C₈-Cycloalkyl, -CF₃, - Halogen, -CO₂(R₃₁), -CON(R₃₁)(R₃₂), -CO(R₃₁), -(CH2)ₙ-N(R₃₁)(CO(R₃₃)), -(CH₂)ₙ-N(R₃₁)(SO₂(R₃₃)), wobei der gesättigte Ring wahlweise substituiert sein kann mit 1, 2 oder 3 -C₁ bis C₆-Alkyl, -C₁ bis C₆-Alkoxy, -OH, -CH₂OH oder -(CH₂)ₙ-N(R₃₁)(R₃₂) oder einem Oxo (=O); und
R₆ ist ausgewählt aus: -H, -OH, -Halogen, -CN, -CF₃, -CO₂(R₆₁), -C(O)R₆₁ oder ―C(O)N(R₆₁)(R₆₂) worin R₆₁ und R₆₂ gleich oder verschieden sind und ausgewählt sind aus:
-H,
C₁ bis C₆-Alkyl,
Phenyl, wahlweise substituiert mit 1, 2 oder 3-Halogen, C₁ bis C₆-Alkyl, C₁ bis C₆-Alkoxy, -CF₃, - OH, -CN,
oder worin R₆₁ und R₆₂ zusammengenommen mit dem anhängenden Stickstoff einen Ring bilden, ausgewählt aus -Pyrrolidinyl, -Piperidinyl, -4-Morpholinyl, -4-Thiomorpholinyl, -4-Piperazinyl oder -4-(C₁-C₆-Alkyl)piperazinyl;
pharmazeutisch duldbare Salze, Hydrate, N-Oxide und Solvate davon;
(XVII) Verbindungen, welche die Struktur haben: 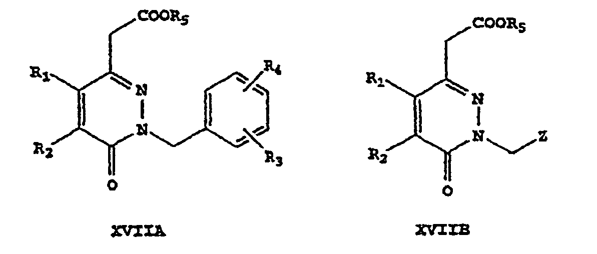
worin R₁ und R₂ H sind, Alkyl, Aryl oder Arylalkyl, wobei das Alkyl als Substituenten Halogen einschließen kann, CF₃, CH₃O, CH₃S, NO₂, oder R₁ und R₂ können mit den Kohlenstoffatomen, an denen sie angebracht sind, Methylendioxy bilden, oder
R₁ und R₂ können einen nichtaromatischen C₃-C₇-Ring bilden oder einen Heterocyclus, der Pyridin sein kann, Pyrazin, Pyrimidin, Pyridazin, Indol oder Pyrazol, oder einen Sauerstoff enthaltenden Heterocyclus, der Pyran sein kann oder Furan, oder einen Schwefel enthaltenden Heterocyclus, der Thiopyran sein kann oder Thiophen; wobei die Heterocyclen wahlweise substituiert sind mit Halogen oder Alkyl;
R₃ und R₄ sind H, Alkyl, Halogen, CF₃, CH₃O, CH₃S oder NO₂, oder R₃ und R₄ bilden mit den Kohlenstoffatomen, an denen sie hängen, eine Methylendioxy-Gruppe;
R₅ ist H und
Z ist ein Heterocyclus, der Pyridin sein kann, Thiazol, Benzothiazol, Benzimidazol oder Chinolin, wobei die Z-Gruppe wahlweise substituiert sein kann mit Halogen oder Alkyl.

15. Verwendung nach Anspruch 1, wobei der aP2-Hemmer die Struktur hat: 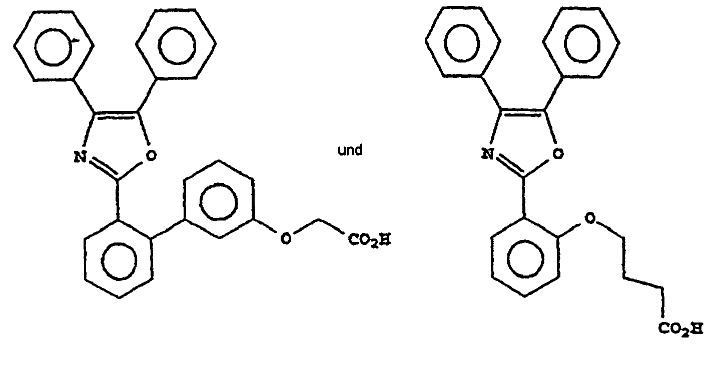 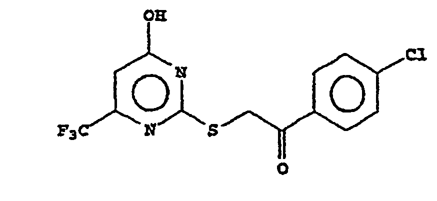 oder 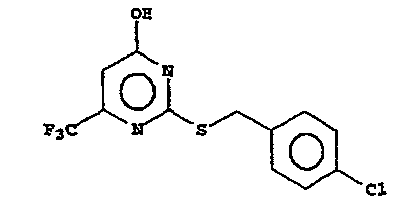 oder

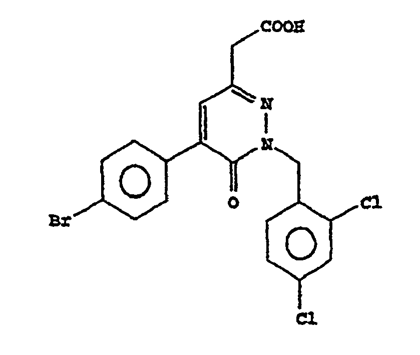

16. Pharmazeutische Kombination, aufweisend einen aP2-Hemmer und ein antidiabetisches Mittel eines anderen Typs.

17. Kombination nach Anspruch 16, wobei das antidiabetische Mittel ein Biguanid ist, ein Sulfonylharnstoff, ein Glucosidase-Hemmer, ein Thiazolidindion, ein Insulin-Sensibilisiermittel, ein Glucagon-ähnliches Peptid-1 (GLP-1), Insulin, ein PPAR α/γ-Dual-Agonist und/oder ein Meglitinid.

18. Kombination nach Anspruch 16, wobei das antidiabetische Mittel Metformin ist, Glyburid, Glimepirid, Glipyrid, Glipizid, Chlorpropamid, Gliclazid, Acarbose, Miglitol, Troglitazon, Insulin, KRP-297, Repaglinid und/oder Nataglinid.

19. Kombination nach Anspruch 16, wobei der aP2-Hemmer in einem Gewichtsverhältnis im Bezug auf das antidiabetische Mittel im Bereich von etwa 0,01 bis etwa 100:1 1 vorliegt.

20. Pharmazeutische Zusammensetzung nach Anspruch 16 zur Behandlung von Insulinresistenz, Diabetes, Obesitas, Hyperglykämie, Hyperinsulinämie oder erhöhte Serumspiegel an freien Fettsäuren oder Glycerin oder Hypertriglyceridämie.

## Revendications

1. Utilisation d'un inhibiteur aP2 pour la préparation d'une composition pharmaceutique pour le traitement du diabète, de la résistance à l'insuline, de l'obésité, de l'hyperglycémie, de l'hyperinsulinémie, ou du taux élevé d'acides gras, ou de glycérol, ou l'hypertriglycéridémie.

2. Utilisation selon la revendication 1, dans laquelle l'inhibiteur aP2 se lie à la protéine aP2 et inhibe sa fonction et/ou sa capacité à se lier aux acides gras libres.

3. Utilisation selon la revendication 1, dans laquelle l'inhibiteur aP2 contient un groupe donneur ou accepteur de liaisons hydrogène et interagit directement ou à travers une molécule d'eau intervenante soit par des interactions ioniques soit de liaisons hydrogène, avec un, deux, ou trois des trois résidus d'acides aminés, désignés par Arg 106, Arg 126 et Tyr 128 dans l'aP2 humaine à l'intérieur de la protéine aP2.

4. Utilisation selon la revendication 3, dans laquelle le groupe donneur ou accepteur de liaisons hydrogène est de nature acide.

5. Utilisation selon la revendication 3, où ledit inhibiteur aP2 contient un substituant supplémentaire qui se lie à (dans) et/ou qui interagit avec une zone distincte à l'intérieur de la protéine aP2 définie grossièrement par les résidus d'acides aminés Phe 16, Tyr 19, Met 20, Val 23, Val 25, Ala 33, Phe 57, Thr 74, Ala 75, Asp 76, Arg 78 dans l'aP2 humaine.

6. Utilisation selon la revendication 5, dans laquelle ledit substituant supplémentaire dans ledit inhibiteur aP2 est de nature hydrophobe.

7. Utilisation selon la revendication 5, dans laquelle la distance dans l'espace entre le groupe donneur/accepteur de liaisons hydrogène et le groupe substituant supplémentaire dans ledit inhibiteur aP2 se situe dans la distance d'environ 7 à environ 15 angströms.

8. Utilisation selon la revendication 1, dans laquelle le diabète de type II est traité.

9. Utilisation selon la revendication 1, dans laquelle l'inhibiteur aP2 est utilisé sous la forme d'un sel pharmaceutiquement acceptable de celui-ci ou d'un ester promédicament de celui-ci.

10. Utilisation selon la revendication 1, dans laquelle l'inhibiteur aP2 inclut un cycle oxazole ou analogue, un dérivé pyrimidine ou un dérivé pyridazinone.

11. Utilisation selon la revendication 10, dans laquelle l'inhibiteur aP2 est un dérivé de benzoyle substitué ou d'acide biphényl-2-oxazole-alcanoïque, un dérivé d'oxazole, un S-dérivé de 2-thio-4,5-diphényloxazole, un dérivé d'oxazole phényl-hétérocyclique, un dérivé de diaryloxazole, un dérivé de 4,5-diphényloxazole, un dérivé d'acide oxazole carboxylique, un dérivé de phényloxazolyloxazole, ou un dérivé d'acide phénoxyalcanoïque substitué par un groupe 2-(4,5-diaryl)-2-oxazolyle.

12. Utilisation selon la revendication 10, dans laquelle l'inhibiteur aP2 est un dérivé de 2-benzyloxypyrimidine, un dérivé de dihydro(alkylthio)(naphtylméthyl)oxypyrimidine, un dérivé de thiouracile, ou un dérivé de pyrimidine-thioalkyle α-substitué ou un dérivé d'éther d'alkyle.

13. Utilisation selon la revendication 10, dans laquelle l'inhibiteur aP2 est un dérivé d'acide pyridazinone acétique.

14. Utilisation selon la revendication 10, dans laquelle l'inhibiteur aP2 est
(I) un dérivé d'acide biphényle alcanoïque ou benzoylbenzène substitué ou ayant la structure:
I A(CH₂)ₙO-B
dans laquelle
A est un groupe de formule 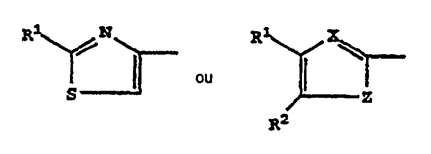 dans laquelle
X est -N- ou 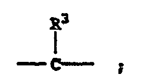 Z est 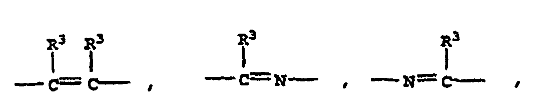 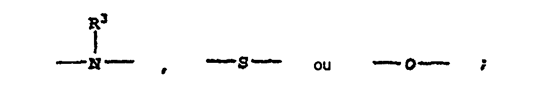 R¹ est un atome d'hydrogène, un groupe alkyle inférieur ou phényle;
R² est un atome d'hydrogène ou un groupe alkyle inférieur; ou
R¹ et R² pris conjointement forment un cycle benzénique, à condition que, lorsque X est -N-, Z soit autre que 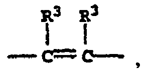 R³ est un atome d'hydrogène ou un groupe alkyle inférieur;
n vaut 1 à 2;
B est 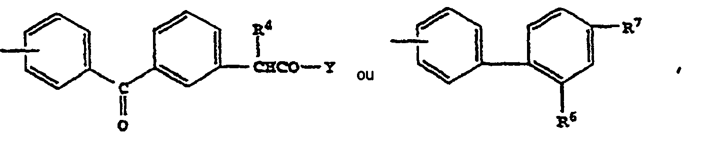 dans lesquelles
Y est OR⁵ ou N(OH)R⁸;
R⁴ et R⁵ sont chacun indépendamment, un atome d'hydrogène ou un groupe alkyle inférieur;
R⁶ est un atome d'hydrogène, un groupe halogéno ou nitro;
R⁷ est 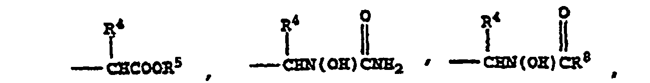 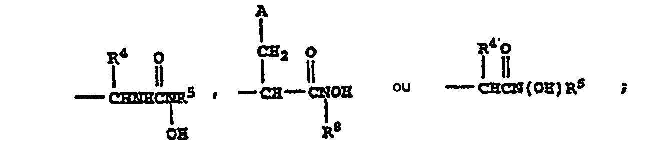 R⁸ est un groupe alkyle inférieur;
m vaut 0 à 3;
ou des sels pharmacologiquement acceptables de celui-ci;
(II) des dérivés oxazole qui ont la structure 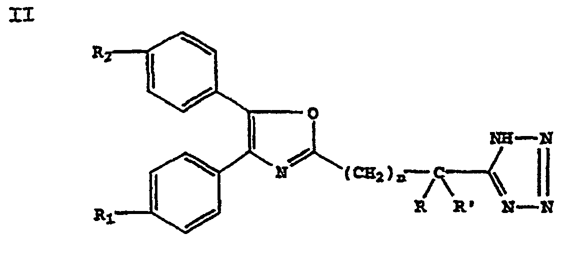 dans laquelle;
R et R' sont identiques ou différents et représentent un atome d'hydrogène ou un radical alkyle contenant 1 ou 2 atome(s) de carbone,
R₁ et R₂ sont identiques ou différents et représentent des atomes d'hydrogène ou d'halogène ou des radicaux alkyloxy dans lesquels la portion alkyle contient 1 à 4 atome(s) de carbone dans une chaîne linéaire ou ramifiée, et
n est égal à 3 à 6, ainsi que leurs sels;
(III) des S-dérivés de 2-thiol-4,5-diphényloxazole qui ont la structure 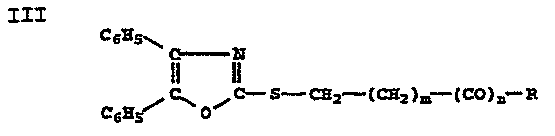 dans laquelle m vaut 0, 1 ou 2, n vaut 1 et R représente un groupe hydroxy, alcoxy ou amino, et des sels d'addition pharmaceutiquement acceptables de ceux-ci;
(IV) des dérivés d'azole de structure 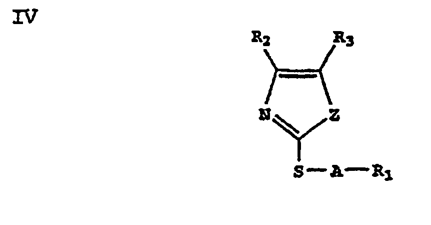 dans laquelle R₁ est un groupe carboxyle, carboxyle estérifié ou un autre groupe carboxyle fonctionnellement modifié; R₂ et R₃ sont chacun un groupe aryle allant jusqu'à 10 atomes de carbone; A représente CₙH₂ₙ dans laquelle n est un nombre entier valant 1 à 10, inclus; et Z est O ou S, et des sels physiologiquement acceptables de ceux-ci;
(V) des dérivés d'oxazole phényl-hétérocyclique qui ont la structure 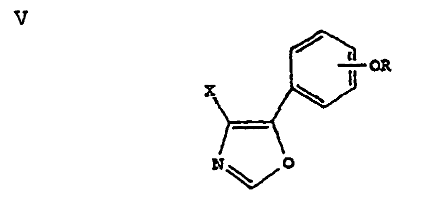 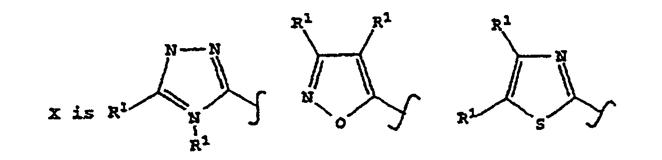 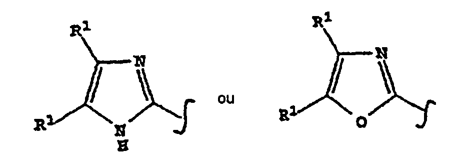 R est CH₂R²;
R¹ est Ph ou Th;
R² est 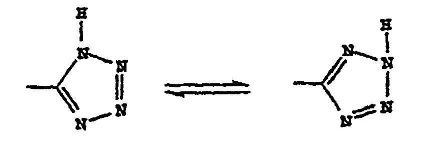 CO₂R³; et
R³ est H, ou un groupe alkyle inférieur en C₁-C₄;
ou un sel pharmaceutiquement acceptable de ceux-ci;
(VI) des dérivés de diaryloxazole ayant la structure 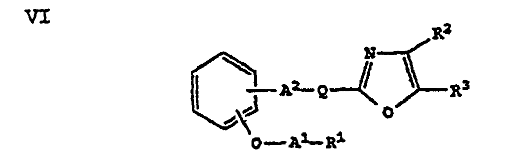 dans laquelle R¹ est un groupe carboxy ou carboxy protégé,
R² est un groupe aryle,
R³ est un groupe aryle,
A¹ est un groupe alcylène inférieur,
A² est une liaison ou un groupe alcylène inférieur et
-Q- est 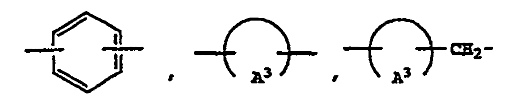 ou 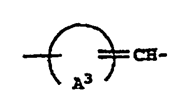 (dans laquelle 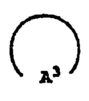 est un groupe cycloalcane (inférieur) ou cycloalcène (inférieur)
dont chacun peut avoir un(des) substituant(s) approprié(s));
(VII) des dérivés de 4,5-diphényloxazole ayant la structure 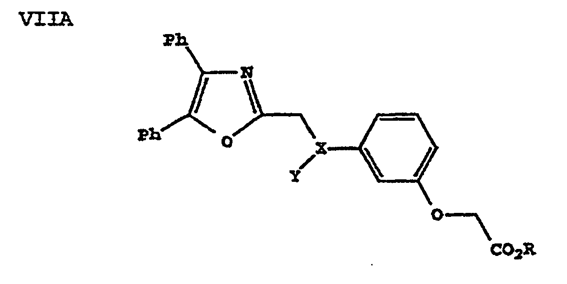 dans laquelle
R est H ou un groupe alkyle inférieur en C₁-C₅,
X est N ou CH,
Y est H ou CO₂R¹, ou COR², à condition que lorsque X est CH, Y ne soit pas H,
R¹ est un groupe alkyle inférieur en C₁-C₅, ou un groupe phénylméthyle, et
R² est un groupe alkyle en C₁-C₅; 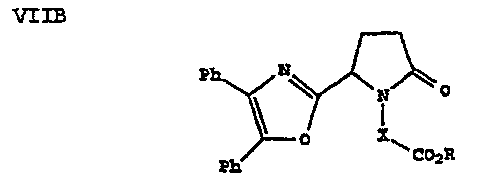 dans laquelle
R est H ou un groupe alkyle inférieur en C₁-C₅,
X est (CH₂)ₙ ou un groupe phényle substitué en position *para* ou *méta* dans lequel le substituant est OR²,
R² est un groupe alkyle en C₁-C₅, et
n est un nombre entier ayant une valeur allant de 4 à 8,
et des sels pharmaceutiquement acceptables de ceux-ci;
(VIII) des dérivés d'acide oxazole carboxylique ayant la structure 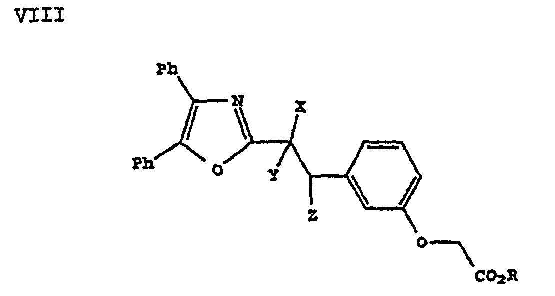 dans laquelle
Y et Z sont indépendamment un atome d'hydrogène ou conjointement forment une liaison;
X est CN, CO₂R¹ ou CONR²R³;
R et R¹ sont indépendamment ou conjointement H, Na, ou un groupe alkyle inférieur en C₁-C₅;
R² et R³ sont indépendamment ou conjointement H, ou un groupe alkyle inférieur en C₁-C₅;
ou un sel de métal alcalin de ceux-ci;
(IX) des dérivés de phényloxazolyloxazole ayant la structure 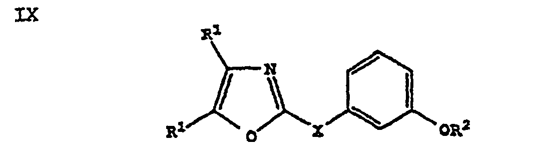 dans laquelle
X est 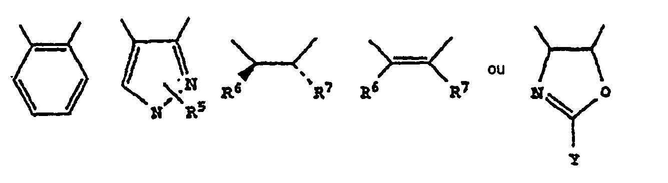 Y est CH₃, Ph, ou OH, à condition que, lorsque Y est OH, le composé existe sous la forme tautomère céto-énol 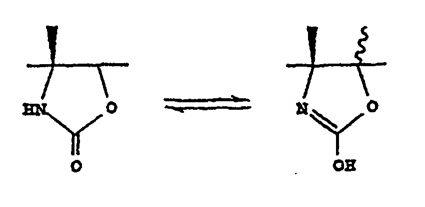 R¹ est Ph ou Th;
R² est CH₂R³;
R³ est CO₂R⁴;
R⁴ est H ou un groupe alkyle inférieur en C₁-C₅;
R⁵ est H ou CH₃; R⁶ est OHCHN ou H₂N; et
R⁷ est H ou OH;
ou un sel pharmaceutiquement acceptable de celui-ci;
(X) des acides phénoxyalcanoïques substitués par un groupe 2-(4,5-diaryl)-2-oxazolyle et des esters ayant la structure 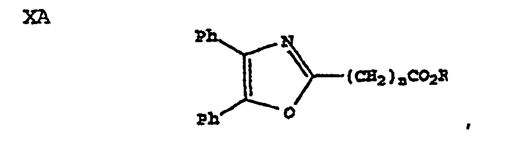 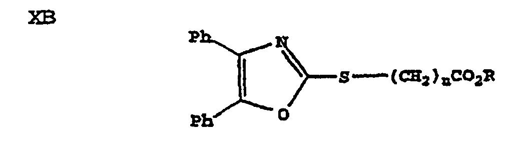 (dans lesquelles n vaut 7 à 9 et R est un atome d'hydrogène ou un groupe alkyle inférieur; ou lorsque R représente un atome d'hydrogène, le sel de métal alcalin de celui-ci), 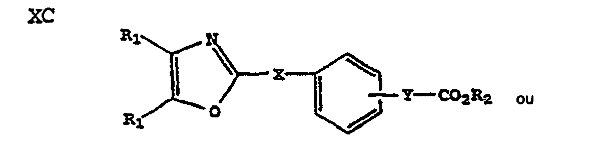 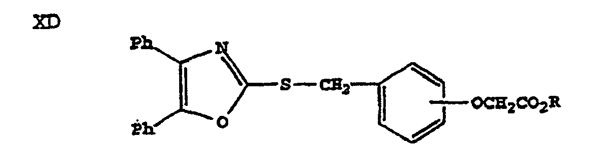 dans laquelle
R₁ est un groupe phényle ou thiényle;
R₂ est un atome d'hydrogène, un groupe alkyle inférieur ou conjointement avec CO₂ est un groupe tétrazol-1-yle;
X est un groupe divalent de connexion choisi parmi le groupe constitué de CH₂CH₂, CH=CH, et CH₂O;
Y est un groupe divalent de connexion fixé à la position 3- ou 4-phényle choisi parmi le groupe constitué de OCH₂, CH₂CH₂ et CH=CH;
ou lorsque R₂ est un atome d'hydrogène, un sel de métal alcalin de celui-ci;
(XI) des hétérocycles 4,5-diaryliques substitués ayant la formule 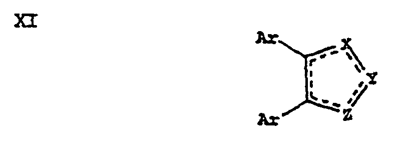 dans laquelle
chaque groupe Ar est identique ou différent et est un groupe phényle éventuellement substitué ou hétéroaryle éventuellement substitué;
X est un atome d'azote ou un groupe CR¹;
Y est un atome d'azote, N(CH₂)ₙA ou C(CH₂)ₙA;
Z est un atome d'azote, un atome d'oxygène ou N(CH₂)ₙA, et la ligne en pointillé indique la présence facultative d'une double liaison afin de former un cycle hétérocyclique complètement insaturé;
R¹ est un atome d'hydrogène, un groupe alkyle en C₁-C₄, un groupe phényle éventuellement substitué ou hétéroaryle éventuellement substitué;
n vaut 4 à 12; et
A vaut CO₂H ou un groupe hydrolysable en CO₂H, un groupe 5-tétrazolyle, SO₃H, P(O)(OR)₂, P(O)(OH)₂, ou P(O)(R)(OR) dans lequel R est un atome d'hydrogène ou un groupe alkyle en C₁-C₄, ou un sel pharmaceutiquement acceptable de celui-ci;
(XII) des composés qui ont la structure 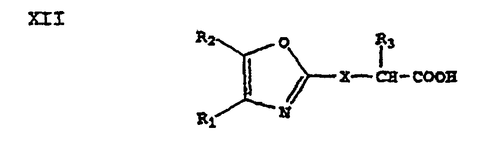 où X représente O ou S;
R₁ représente H, un groupe phényle ou phényle substitué par F, Cl ou Br ou un groupe alcoxy,
R₂ est H, un groupe alkyle, phényle ou phényle substitué par F, Cl ou Br ou un groupe alcoxy, et
R₃ est H ou un groupe alkyle;
(XIII) des dérivés de 2-benzyloxypyrimidine ayant la structure suivante 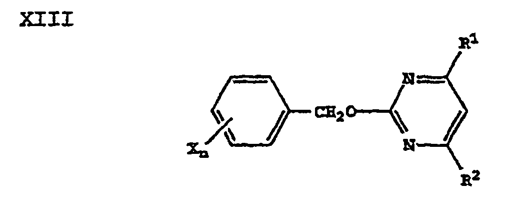 dans laquelle
R₁ et R₂ sont chacun indépendamment H, un atome d'halogène, un groupe hydroxyle, alkyle en C₁-C₄, halogénoalkyle en C₁-C₄, alcényle en C₃-C₅, alcynyle en C₃-C₅, alcoxy en C₁-C₄, halogénoalcoxy en C₁-C₄, alcényloxy en C₃-C₅, alcynyloxy en C₃-C₅, alkylthio en C₁-C₄, ou phényle, à condition qu'au moins l'un parmi R¹ et R² puisse être un groupe hydroxyle;
n est un nombre entier ayant une valeur allant de 0 à 5; et
chaque X qui peut être identique ou différent si n est supérieur à 1, est un atome d'halogène, un groupe alkyle en C₁-C₄, halogénoalkyle en C₁-C₄, alcoxy en C₁-C₄, alkylthio en C₁-C₄, aralkyloxy en C₇-C₉, phényle, hydroxyméthyle, hydroxycarbonyle, alcoxycarbonyle en C₁-C₄, ou nitro;
(XIV) des dihydro(alkylthio)-(naphtylméthlyl)-oxopyrimidines qui ont les structures 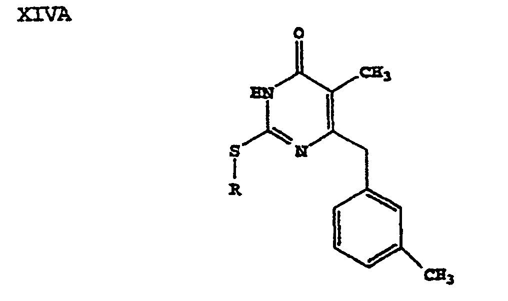 3a R = sec-butyle
3b R = cyclopentyle
3c R = cyclohexyle 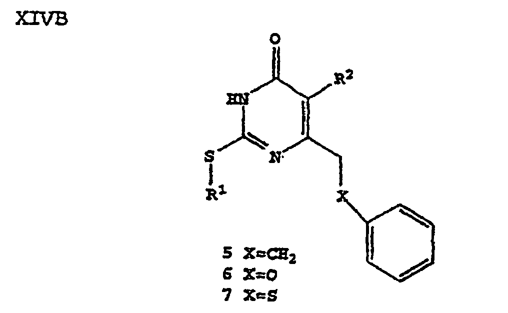 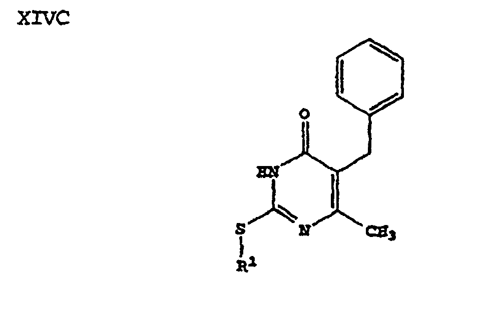 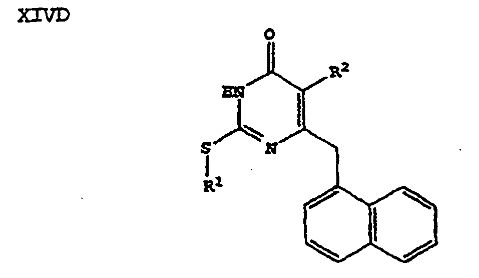 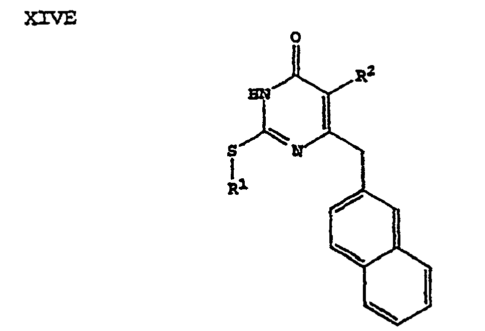 R¹ = sec-butyle, cyclopentyle, cyclohexyle;
R² = H, CH₃, incluant les tautomères de ceux ci-dessus;
(XVI) des composés pyrimidine-thioalkyle α-substitué et alkyléther qui ont la structure 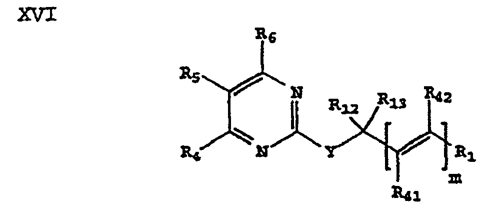 où m vaut 0 ou 1;
R¹ est choisi parmi -CO₂R₅₃, -CONR₅₄R₅₅, 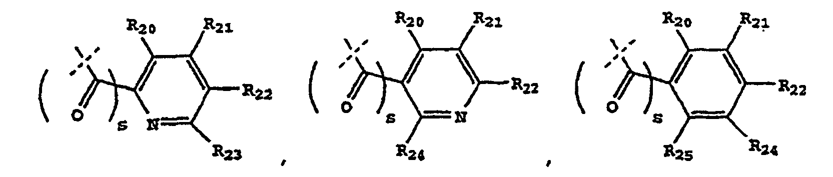 où s vaut 0 ou 1, et R₂₀, R₂₁, R₂₂, R₂₃, R₂₄, et R₂₅ sont identiques ou différents et sont choisis parmi -H, un groupe alkyle en C₁-C₆, alcényle en C₁-C₆, alcoxy en C₁-C₆, alkylthio en C₁-C₆, cycloalkyle en C₃-C₈, -CF₃, -NO₂, -halogéno, -OH, -CN, phényle, phénylthio, styryle, -CO₂(R₃₁), -CON(R₃₁)(R₃₂), -CO(R₃₁), - (CH₂)ₙ-N(R₃₁)(R₃₂), -C(OH)(R₃₁)(R₃₃), -(CH2)ₙN(R₃₁)(CO(R₃₃)), (CH₂)ₙN(R₃₁)(SO₂(R₃₃)), ou dans laquelle R₂₀ et R₂₁, ou R₂₁ et R₂₂, ou R₂₂ et R₂₃ sont pris conjointement pour former un cycle saturé ou insaturé à cinq ou six chaînons contenant 0 ou 1 atome d'oxygène, d'azote ou de soufre, où le cycle insaturé peut être éventuellement substitué par 1, 2 ou 3, groupe alkyle en C₁-C₆, alcoxy en C₁-C₆, -OH, -CH₂OH, -(CH₂)ₙ-N(R₃₁)(R₃₂), cycloalkyle en C₃-C₈, -CF₃, -halogéno, CO₂(R₃₁), -CON(R₃₁)(R₃₂), -CO(R₃₁), - (CH2)ₙN(R₃₁)(CO(R₃₃)), -(CH₂)ₙN(R₃₁)(SO₂(R₃₃)), -CN, -CH₂CF₃ ou -CH(CF₃)₂, ou phényle et le cycle saturé peut être éventuellement substitué par 1, 2 ou 3, groupe alkyle en -C₁-C₆, alcoxy en -C₁-C₆, -OH, - CH₂OH ou -(CH₂)n-N(R₃₁)(R₃₂) ou un groupe oxo (=O);
dans laquelle n vaut 0 à 3 et R₃₁, R₃₂ et R₃₃ sont identiques ou différents et sont choisis parmi
-H,
un groupe alkyle en C₁-C₆,
phényle éventuellement substitué par 1, 2 ou 3 groupe(s) -halogéno, alkyle en C₁-C₆, alcoxy en C₁-C₆, -CF₃, -OH ou -CN,
ou dans laquelle R₃₁ et R₃₂ pris conjointement avec l'atome d'azote fixé pour former un cycle choisi parmi le groupe -pyrrolidinyle, -pipéridinyle, -4-morpholinyle, -4-thiomorpholinyle, -4-pipérazinyle, -4-(1-alkyle en C₁-C₆)pipérazinyle, ou un membre choisi parmi
le groupe 1-cyclohexényle, 2-pyrimidinyle, 4-pyrimidinyle, 5-pyrimidinyle, 2-imidazolyle, 4-imidazolyle, 2-benzothiazolyle, 2-benzoxazolyle, 2-benzimidazolyle, 2-oxazolyle, 4-oxazolyle, 2-thiazolyle, 3-isoxazolyle, 5-isoxazolyle, 5-méthyl-3-isoxazolyle, 5-phényl-3-isoxazolyle, 4-thiazolyle, 3-méthyl-2-pyrazinyle, 5-méthyl-2-pyrazinyle, 6-méthyl-2-pyrazinyle, 5-chloro-2-thiényle, 3-furyle, benzofuran-2-yle, benzothién-2-yle, 2H-1-benzopyran-3-yle, 2,3-dihydrobenzopyran-5-yle, 1-méthylimidazol-2-yle, quinoxalin-2-yle, pipéron-5-yle, 4,7-dichlorobenzoxazol-2-yle, 4,6-diméthylpyrimidin-2-yle, 4-méthylpyrimidin-2-yle, 2,4-diméthylpyrimidin-6-yle, 2-méthylpyrimidin-4-yle, 4-méthylpyrimidin-6-yle, 6-chloropipéron-5-yle, 5-chloroimidazol[1,2-a]pyridin-2-yle, 1-H-indén-3-yle, 1-H-2-méthyl-indén-2-yle, 3,4-dihydronapht-1-yle, S-4-isopropénylcyclohexén-1-yle ou 4-dihydronapht-2-yle;
dans laquelle R₅₃ est choisi parmi -H, un groupe alkyle en C₁-C₆, cycloalkyle en C₃-C₆, phényle (éventuellement substitué par 1, 2, ou 3 groupe(s) -halogéno, alkyle en C₁-C₆, alcoxy en C₁-C₆, -CF₃, -OH, - CN), ou un cycle insaturé à cinq ou six chaînons contenant 0 ou 1 atome d'oxygène, d'azote ou de soufre, où le cycle insaturé peut être éventuellement substitué par -H, un groupe alkyle en C₁-C₆, alcoxy en C₁-C₆, - OH, -CH₂OH, ou -(CH₂)ₙ-N(R₃₁)(R₃₂); où R₅₄ et R₅₅ étant identiques ou différents sont choisis parmi -H, un groupe alkyle en C₁-C₆, allyle, ou phényle (éventuellement substitué par 1, 2 ou 3 groupe(s) -halogéno, alkyle en C₁-C₆, alcoxy en C₁-C₆ ou -CF3), ou pris conjointement avec l'atome d'azote fixé pour former un cycle choisi parmi un groupe -pyrrolidinyle, -pipéridinyle, -4-morpholinyle, -4-thiomorpholinyle, -4-pipérazinyle, -4-(1-alkyle en C₁-C₆)pipérazinyle;
R₄₁ et R₄₂, étant identiques ou différents, sont choisis parmi -H et un groupe alkyle en C₁-C₄;
R₁₂ est choisi parmi -H, un groupe alkyle en C₁-C₆, cycloalkyle en C₃-C₆, -CN, -C(O)NH₂, - C(O)N(alkyle en C₁-C₆)(alkyle en C₁-C₆), CO₂H, -CO₂(alkyle en C₁-C₆), -CH₂OH, -CH₂NH₂ ou -CF₃;
R₁₃ est choisi parmi -H, un groupe alkyle en C₁-C₆ ou -CF₃;
Y est choisi parmi -S-, -S(O)-, -S(O)₂, ou -O-;
R₄ est -OH;
R₅ est choisi parmi le groupe -H, -C₂H₄OH, -C₂H₄-O-TBDMS, halogéno, cycloalkyle en C₃-C₆, alcoxy en C₁-C₃, -CH₂CH₂Cl ou alkyle en C₁-C₄, à condition que R₅ ne soit pas un groupe isobutyle;
ou, lorsque R₆ est un groupe hydroxyle, R₄ et R₅ sont pris conjointement pour former un cycle saturé ou insaturé à cinq ou six chaînons qui conjointement avec le cycle pyrimidine forment le groupe constitué de la 7H-pyrrolo[2,3-d]pyrimidine, 5,6-dihydro-7H-pyrrolo[2,3-d]pyrimidine, furo[2,3-d]pyrimidine, 5,6-dihydro-furo[2,3-d]pyrimidine, thiéno[2,3-d]pyrimidine, 5,6-dihydro-thiéno[2,3-d]pyrimidine, 1H-pyrazolo[3,4-d]pyrimidine, 1H-purine, pyrimido[4,5-d]pyrimidine, ptéridine, pyrido[2,3-d]pyrimidine, ou de la quinazoline, où le cycle insaturé peut être éventuellement substitué par 1, 2 ou 3, groupe alkyle en C₁-C₆, alcoxy en C₁-C₆, -OH, -CH₂OH, ou -(CH₂)ₙ-N(R₃₁)(R₃₂), cycloalkyle en C₃-C₈, - CF₃, -halogéno, -CO₂(R₃₁), -CON(R₃₁)(R₃₂), -CO(R₃₁), -(CH₂)ₙN(R₃₁)(CO(R₃₃)), -(CH₂)ₙN(R₃₁)(SO₂(R₃₃)), et le cycle saturé peut être éventuellement substitué par 1, 2 ou 3, groupe(s) alkyle en C₁-C₆, alcoxy en C₁-C₆, -OH, -CH₂OH, ou -(CH₂)ₙ-N(R₃₁)(R₃₂) ou un groupe oxo (=O);
et
R₆ est choisi parmi -H, -OH, halogéno, -CN, -CF₃, -CO₂(R₆₁), -C(O)R₆₁ ou -C(O)N(R₆₁)(R₆₂) où R₆, et R₆₂ sont identiques ou différents et sont choisis parmi
-H
un groupe alkyle en C₁-C₆,
un groupe phényle éventuellement substitué par 1, 2 ou 3 groupe(s) -halogéno, alkyle en C₁-C₆, alcoxy en C₁-C₆, -CF₃, -OH, -CN,
ou dans laquelle R₆, et R₆₂ pris conjointement avec l'azote fixé pour former un cycle choisi parmi le -pyrrolidinyle, -pipéridinyle, -4-morpholinyle, -4-thiomorpholinyle, -4-pipérazinyle, ou -4-(alkyle en C₁-C₆)pipérazinyle;
les sels pharmaceutiquement acceptable, hydrates, N-oxides et solvates de ceux-ci;
(XVII) des composés qui ont la structure 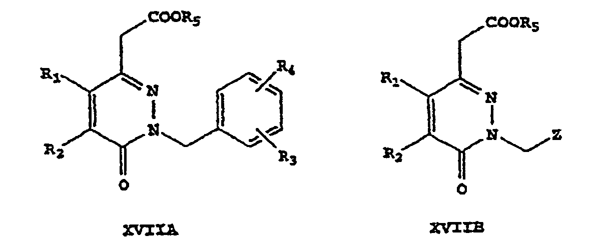
où R₁ et R₂ sont H, un groupe alkyle, aryle ou arylalkyle, où le groupe alkyle peut inclure comme substituants un groupe halogéno, CF₃, CH₃O, CH₃S, NO₂, ou R₁ et R₂ avec les atomes auxquels ils sont fixés peuvent former un groupe méthylènedioxy, ou
R₁ et R₂ peuvent former un cycle non aromatique en C₃-C₇, ou un hétérocycle qui peut être la pyridine, la pyrazine, la pyrimidine, la pyridazine, l'indol, ou le pyrazole, ou un hétérocycle contenant un oxygène qui peut être le pyrane ou le furane, ou un hétérocycle contenant du soufre qui peut être le thiopyrane ou le thiophène; les hétérocycles étant éventuellement substitués par un groupe halogéno ou un groupe alkyle,
R₃ et R₄ sont H, un groupe alkyle, halogéno, CF₃, CH₃O, CH₃S ou NO₂ ou R₃ et R₄ avec les atomes auxquels ils sont fixés peuvent former un groupe méthylènedioxy,
R₅ est H, et
Z est un hétérocycle qui peut être un groupe pyridine, thiazole, benzothiazole, benzimidazole ou quinoléine, lequel groupe Z peut éventuellement être substitué par un groupe halogéno ou alkyle.

15. Utilisation selon la revendication 1, dans laquelle l'inhibiteur aP2 a la structure 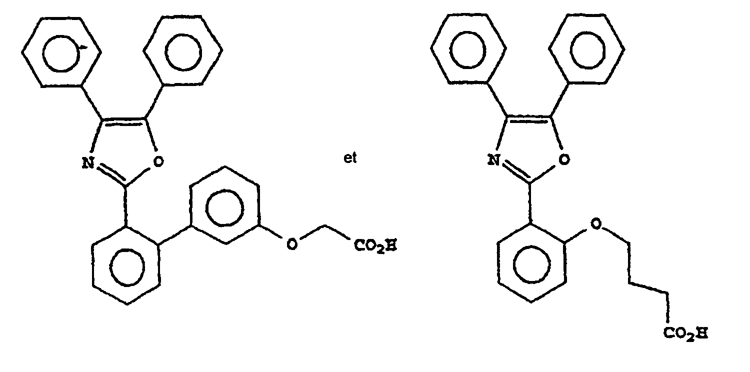 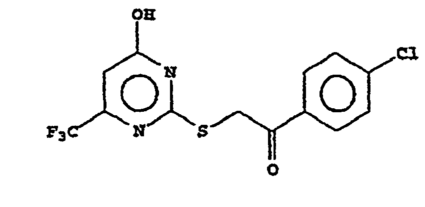 ou 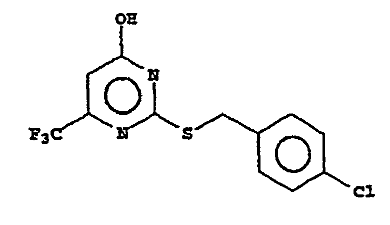 ou

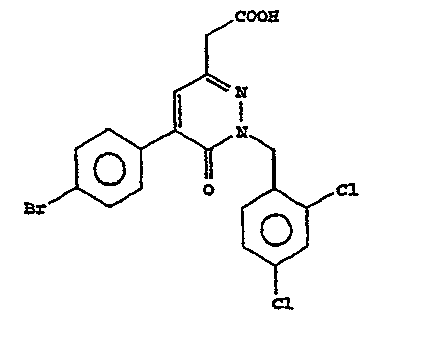

16. Combinaison pharmaceutique comprenant un inhibiteur aP2 et un autre type d'agent antidiabétique.

17. Combinaison selon la revendication 16, dans laquelle l'agent antidiabétique est un biguanide, une sulfonyl urée, un inhibiteur de la glucosidase, une thiazolidinedione, un agent de sensibilisation à l'insuline, un peptide semblable au glucagon-1 (GLP-1), l'insuline, un agoniste double PPAR α/γ et/ou un méglitinide.

18. Combinaison selon la revendication 16, dans laquelle l'agent antidiabétique est le metformin, le glyburide, le glimépiride, le glipyride, le glipizide, le chlorpropamide, le gliclazide, l'acarbose, le miglitol, le troglitazone, l'insuline, le KRP-297, le répaglinide et/ou le nataglinide.

19. Combinaison selon la revendication 16, dans laquelle l'inhibiteur aP2 est présent en un rapport en poids par rapport à l'agent antidiabétique compris dans la plage d'environ 0,01 à environ 100: 1.

20. Composition pharmaceutique selon la revendication 16 pour traiter la résistance à l'insuline, le diabète, l'obésité, l'hyperglycémie, l'hyperinsulinémie, ou les concentrations sanguines élevées d'acides gras libres, ou de glycérol, ou l'hypertriglycéridémie.
